# EUROPEAN PATENT SPECIFICATION

(11) **EP 3 360 958 B1**
(45) Date of publication and mention of the grant of the patent: **22.12.2021**
(21) Application number: 17206830.6
(22) Date of filing: 15.03.2014
(51) Int. Cl.: C12N 9/00, C12Q 1/68, C12P 19/34

(54) **THERMOSTABLE BLUNT-END LIGASE AND METHODS OF USE**
WÄRMESTABILE OFFENENDIGE LIGASE UND VERFAHREN ZUR VERWENDUNG
LIGASE À EXTRÉMITÉ FRANCHE THERMOSTABLE ET PROCÉDÉS D'UTILISATION

(30) Priority: 15.03.2013 US 201361802124 P
(43) Date of publication of application: 15.08.2018
(62) Divisional of application: 14762285.6
(73) Proprietor: Labrador Diagnostics LLC, Wilmington, Delaware 19801 (US)
(72) Inventor: CHRISTIANS, Frederick, Palo Alto, AZ Arizona CA 94304 (US)
(74) Representative: EIP

(56) References cited:
- WO-A1-94/02615
- WO-A1-2011/034449
- WO-A1-2015/076919
- G Lauer ET AL: "Cloning, nucleotide sequence, and engineered expression of Thermus thermophilus DNA ligase, a homolog of Escherichia coli DNA ligase", Journal of Bacteriology, 1 August 1991 (1991-08-01), pages 5047-5053, XP055284088, UNITED STATES Retrieved from the Internet: URL:http://jb.asm.org/content/173/16/5047. full.pdf#page=1&view=FitH [retrieved on 2016-08-26]

### SEQUENCE LISTING

<110> THERANOS, INC.
<120> THERMOSTABLE BLUNT-END LIGASE AND METHODS OF USE
<140> EP 14 762285.6
   <141> 2014-03-15
<150> US 61/802,124
   <151> 2013-03-15
<160> 23
<170> Patent In version 3.5
<210> 1
   <211> 1038
   <212> PRT
   <213> Artificial Sequence
<220>
   <223> Description of Artificial Sequence: Synthetic polypeptide
<400> 1
   Met Gly His His His His His His His His His His Ser Ser Gly His
   1               5                   10                  15
   Ile Glu Gly Arg Ala Ser Ala Asp Gly Pro Tyr Leu Gln Ile Leu Glu
               20                  25                  30
   Gln Pro Lys Gln Arg Gly Phe Arg Phe Arg Tyr Val Cys Glu Gly Pro
           35                  40                  45
   Ser His Gly Gly Leu Pro Gly Ala Ser Ser Glu Lys Asn Lys Lys Ser
       50                  55                  60
   Tyr Pro Gln Val Lys Ile Cys Asn Tyr Val Gly Pro Ala Lys Val Ile
   65                  70                  75                  80
   Val Gln Leu Val Thr Asn Gly Lys Asn Ile His Leu His Ala His Ser
                   85                  90                  95
   Leu Val Gly Lys His Cys Glu Asp Gly Ile Cys Thr Val Thr Ala Gly
               100                 105                 110
   Pro Lys Asp Met Val Val Gly Phe Ala Asn Leu Gly Ile Leu His Val
           115                 120                 125
   Thr Lys Lys Lys Val Phe Glu Thr Leu Glu Ala Arg Met Thr Glu Ala
       130                 135                 140
   Cys Ile Arg Gly Tyr Asn Pro Gly Leu Leu Val His Pro Asp Leu Ala
   145 150 155 160
   Tyr Leu Gln Ala Glu Gly Gly Gly Asp Arg Gln Leu Gly Asp Arg Glu
   165 170 175
   Lys Glu Leu Ile Arg Gln Ala Ala Leu Gln Gln Thr Lys Glu Met Asp
   180 185 190
   Leu Ser Val Val Arg Leu Met Phe Thr Ala Phe Leu Pro Asp Ser Thr
   195 200 205
   Gly Ser Phe Thr Arg Arg Leu Glu Pro Val Val Ser Asp Ala Ile Tyr
   210 215 220
   Asp Ser Lys Ala Pro Asn Ala Ser Asn Leu Lys Ile Val Arg Met Asp
   225 230 235 240
   Arg Thr Ala Gly Cys Val Thr Gly Gly Glu Glu Ile Tyr Leu Leu Cys
   245 250 255
   Asp Lys Val Gln Lys Asp Asp Ile Gln Ile Arg Phe Tyr Glu Glu Glu
   260 265 270
   Glu Asn Gly Gly Val Trp Glu Gly Phe Gly Asp Phe Ser Pro Thr Asp
   275 280 285
   Val His Arg Gln Phe Ala Ile Val Phe Lys Thr Pro Lys Tyr Lys Asp
   290 295 300
   Ile Asn Ile Thr Lys Pro Ala Ser Val Phe Val Gln Leu Arg Arg Lys
   305 310 315 320
   Ser Asp Leu Glu Thr Ser Glu Pro Lys Pro Phe Leu Tyr Tyr Pro Glu
   325 330 335
   Ile Lys Asp Lys Glu Glu Val Gln Arg Lys Arg Gln Lys Gly Ser Ser
   340 345 350
   Gly Thr Ser Gly Gly Gly Ser Gly Gly Gly Met Thr Leu Glu Glu Ala
   355 360 365
   Arg Lys Arg Val Asn Glu Leu Arg Asp Leu Ile Arg Tyr His Asn Tyr
   370 375 380
   Arg Tyr Tyr Val Leu Ala Asp Pro Glu Ile Ser Asp Ala Glu Tyr Asp
   385 390 395 400
   Arg Leu Leu Arg Glu Leu Lys Glu Leu Glu Glu Arg Phe Pro Glu Leu
   405 410 415
   Lys Ser Pro Asp Ser Pro Thr Leu Gln Val Gly Ala Arg Pro Leu Glu
   420 425 430
   Ala Thr Phe Arg Pro Val Arg His Pro Thr Arg Met Tyr Ser Leu Asp
   435 440 445
   Asn Ala Phe Asn Leu Asp Glu Leu Lys Ala Phe Glu Glu Arg Ile Glu
   450 455 460
   Arg Ala Leu Gly Arg Lys Gly Pro Phe Ala Tyr Thr Val Glu His Lys
   465 470 475 480
   Val Asp Gly Leu Ser Val Asn Leu Tyr Tyr Glu Glu Gly Val Leu Val
   485 490 495
   Tyr Gly Ala Thr Arg Gly Asp Gly Glu Val Gly Glu Glu Val Thr Gln
   500 505 510
   Asn Leu Leu Thr Ile Pro Thr Ile Pro Arg Arg Leu Lys Gly Val Pro
   515 520 525
   Glu Arg Leu Glu Val Arg Gly Glu Val Tyr Met Pro Ile Glu Ala Phe
   530 535 540
   Leu Arg Leu Asn Glu Glu Leu Glu Glu Arg Gly Glu Arg Ile Phe Lys
   545 550 555 560
   Asn Pro Arg Asn Ala Ala Ala Gly Ser Leu Arg Gln Lys Asp Pro Arg
   565 570 575
   Ile Thr Ala Lys Arg Gly Leu Arg Ala Thr Phe Tyr Ala Leu Gly Leu
   580 585 590
   Gly Leu Glu Glu Val Glu Arg Glu Gly Val Ala Thr Gln Phe Ala Leu
   595 600 605
   Leu His Trp Leu Lys Glu Lys Gly Phe Pro Val Glu His Gly Tyr Ala
   610 615 620
   Arg Ala Val Gly Ala Glu Gly Val Glu Ala Val Tyr Gln Asp Trp Leu
   625 630 635 640
   Lys Lys Arg Arg Ala Leu Pro Phe Glu Ala Asp Gly Val Val Val Lys
   645 650 655
   Leu Asp Glu Leu Ala Leu Trp Arg Glu Leu Gly Tyr Thr Ala Arg Ala
               660                 665                 670
   Pro Arg Phe Ala Ile Ala Tyr Lys Phe Pro Ala Glu Glu Lys Glu Thr
           675                 680                 685
   Arg Leu Leu Asp Val Val Phe Gln Val Gly Arg Thr Gly Arg Val Thr
       690                 695                 700
   Pro Val Gly Ile Leu Glu Pro Val Phe Leu Glu Gly Ser Glu Val Ser
   705                 710                 715                 720
   Arg Val Thr Leu His Asn Glu Ser Tyr Ile Glu Glu Leu Asp Ile Arg
                   725                 730                 735
   Ile Gly Asp Trp Val Leu Val His Lys Ala Gly Gly Val Ile Pro Glu
               740                 745                 750
   Val Leu Arg Val Leu Lys Glu Arg Arg Thr Gly Glu Glu Arg Pro Ile
           755                 760                 765
   Arg Trp Pro Glu Thr Cys Pro Glu Cys Gly His Arg Leu Leu Lys Glu
       770                 775                 780
   Gly Lys Val His Arg Cys Pro Asn Pro Leu Cys Pro Ala Lys Arg Phe
   785                 790                 795                 800
   Glu Ala Ile Arg His Phe Ala Ser Arg Lys Ala Met Asp Ile Gln Gly
                   805                 810                 815
   Leu Gly Glu Lys Leu Ile Glu Arg Leu Leu Glu Lys Gly Leu Val Lys
               820                 825                 830
   Asp Val Ala Asp Leu Tyr Arg Leu Arg Lys Glu Asp Leu Val Gly Leu
           835                 840                 845
   Glu Arg Met Gly Glu Lys Ser Ala Gln Asn Leu Leu Arg Gln Ile Glu
       850                 855                 860
   Glu Ser Lys Lys Arg Gly Leu Glu Arg Leu Leu Tyr Ala Leu Gly Leu
   865                 870                 875                 880
   Pro Gly Val Gly Glu Val Leu Ala Arg Asn Leu Ala Ala Arg Phe Gly
                   885                 890                 895
   Asn Met Asp Arg Leu Leu Glu Ala Ser Leu Glu Glu Leu Leu Glu Val
               900                 905                 910
   Glu Glu Val Gly Glu Leu Thr Ala Arg Ala Ile Leu Glu Thr Leu Lys
   915 920 925
   Asp Pro Ala Phe Arg Asp Leu Val Arg Arg Leu Lys Glu Ala Gly Val
   930 935 940
   Glu Met Glu Ala Lys Glu Lys Gly Gly Glu Ala Leu Lys Gly Leu Thr
   945 950 955 960
   Phe Val Ile Thr Gly Glu Leu Ser Arg Pro Arg Glu Glu Val Lys Ala
   965 970 975
   Leu Leu Arg Arg Leu Gly Ala Lys Val Thr Asp Ser Val Ser Arg Lys
   980 985 990
   Thr Ser Tyr Leu Val Val Gly Glu Asn Pro Gly Ser Lys Leu Glu Lys
   995 1000 1005
   Ala Arg Ala Leu Gly Val Pro Thr Leu Thr Glu Glu Glu Leu Tyr
   1010 1015 1020
   Arg Leu Leu Glu Ala Arg Thr Gly Lys Lys Ala Glu Glu Leu Val
   1025 1030 1035
<210> 2
   <211> 327
   <212> PRT
   <213> Homo sapiens
<400> 2
   Ala Asp Gly Pro Tyr Leu Gln Ile Leu Glu Gln Pro Lys Gln Arg Gly
   1 5 10 15
   Phe Arg Phe Arg Tyr Val Cys Glu Gly Pro Ser His Gly Gly Leu Pro
   20 25 30
   Gly Ala Ser Ser Glu Lys Asn Lys Lys Ser Tyr Pro Gln Val Lys Ile
   35 40 45
   Cys Asn Tyr Val Gly Pro Ala Lys Val Ile Val Gln Leu Val Thr Asn
   50 55 60
   Gly Lys Asn Ile His Leu His Ala His Ser Leu Val Gly Lys His Cys
   65 70 75 80
   Glu Asp Gly Ile Cys Thr Val Thr Ala Gly Pro Lys Asp Met Val Val
   85 90 95
   Gly Phe Ala Asn Leu Gly Ile Leu His Val Thr Lys Lys Lys Val Phe
               100                 105                 110
   Glu Thr Leu Glu Ala Arg Met Thr Glu Ala Cys Ile Arg Gly Tyr Asn
           115                 120                 125
   Pro Gly Leu Leu Val His Pro Asp Leu Ala Tyr Leu Gln Ala Glu Gly
       130                 135                 140
   Gly Gly Asp Arg Gln Leu Gly Asp Arg Glu Lys Glu Leu Ile Arg Gln
   145                 150                 155                 160
   Ala Ala Leu Gln Gln Thr Lys Glu Met Asp Leu Ser Val Val Arg Leu
                   165                 170                 175
   Met Phe Thr Ala Phe Leu Pro Asp Ser Thr Gly Ser Phe Thr Arg Arg
               180                 185                 190
   Leu Glu Pro Val Val Ser Asp Ala Ile Tyr Asp Ser Lys Ala Pro Asn
           195                 200                 205
   Ala Ser Asn Leu Lys Ile Val Arg Met Asp Arg Thr Ala Gly Cys Val
       210                 215                 220
   Thr Gly Gly Glu Glu Ile Tyr Leu Leu Cys Asp Lys Val Gln Lys Asp
   225                 230                 235                 240
   Asp Ile Gln Ile Arg Phe Tyr Glu Glu Glu Glu Asn Gly Gly Val Trp
                   245                 250                 255
   Glu Gly Phe Gly Asp Phe Ser Pro Thr Asp Val His Arg Gln Phe Ala
               260                 265                 270
   Ile Val Phe Lys Thr Pro Lys Tyr Lys Asp Ile Asn Ile Thr Lys Pro
           275                 280                 285
   Ala Ser Val Phe Val Gln Leu Arg Arg Lys Ser Asp Leu Glu Thr Ser
       290                 295                 300
   Glu Pro Lys Pro Phe Leu Tyr Tyr Pro Glu Ile Lys Asp Lys Glu Glu
   305                 310                 315                 320
   Val Gln Arg Lys Arg Gln Lys
                   325
<210> 3
   <211> 676
   <212> PRT
   <213> Thermus thermophilus
<220>
   <223> Strain HB8
<400> 3
   Met Thr Leu Glu Glu Ala Arg Lys Arg Val Asn Glu Leu Arg Asp Leu
   1               5                   10                  15
   Ile Arg Tyr His Asn Tyr Arg Tyr Tyr Val Leu Ala Asp Pro Glu Ile
               20                  25                  30
   Ser Asp Ala Glu Tyr Asp Arg Leu Leu Arg Glu Leu Lys Glu Leu Glu
           35                  40                  45
   Glu Arg Phe Pro Glu Leu Lys Ser Pro Asp Ser Pro Thr Leu Gln Val
       50                  55                  60
   Gly Ala Arg Pro Leu Glu Ala Thr Phe Arg Pro Val Arg His Pro Thr
   65                  70                  75                  80
   Arg Met Tyr Ser Leu Asp Asn Ala Phe Asn Leu Asp Glu Leu Lys Ala
                   85                  90                  95
   Phe Glu Glu Arg Ile Glu Arg Ala Leu Gly Arg Lys Gly Pro Phe Ala
               100                 105                 110
   Tyr Thr Val Glu His Lys Val Asp Gly Leu Ser Val Asn Leu Tyr Tyr
           115                 120                 125
   Glu Glu Gly Val Leu Val Tyr Gly Ala Thr Arg Gly Asp Gly Glu Val
       130                 135                 140
   Gly Glu Glu Val Thr Gln Asn Leu Leu Thr Ile Pro Thr Ile Pro Arg
   145                 150                 155                 160
   Arg Leu Lys Gly Val Pro Glu Arg Leu Glu Val Arg Gly Glu Val Tyr
                   165                 170                 175
   Met Pro Ile Glu Ala Phe Leu Arg Leu Asn Glu Glu Leu Glu Glu Arg
               180                 185                 190
   Gly Glu Arg Ile Phe Lys Asn Pro Arg Asn Ala Ala Ala Gly Ser Leu
           195                 200                 205
   Arg Gln Lys Asp Pro Arg Ile Thr Ala Lys Arg Gly Leu Arg Ala Thr
       210                 215                 220
   Phe Tyr Ala Leu Gly Leu Gly Leu Glu Glu Val Glu Arg Glu Gly Val
   225 230 235 240
   Ala Thr Gln Phe Ala Leu Leu His Trp Leu Lys Glu Lys Gly Phe Pro
   245 250 255
   Val Glu His Gly Tyr Ala Arg Ala Val Gly Ala Glu Gly Val Glu Ala
   260 265 270
   Val Tyr Gln Asp Trp Leu Lys Lys Arg Arg Ala Leu Pro Phe Glu Ala
   275 280 285
   Asp Gly Val Val Val Lys Leu Asp Glu Leu Ala Leu Trp Arg Glu Leu
   290 295 300
   Gly Tyr Thr Ala Arg Ala Pro Arg Phe Ala Ile Ala Tyr Lys Phe Pro
   305 310 315 320
   Ala Glu Glu Lys Glu Thr Arg Leu Leu Asp Val Val Phe Gln Val Gly
   325 330 335
   Arg Thr Gly Arg Val Thr Pro Val Gly Ile Leu Glu Pro Val Phe Leu
   340 345 350
   Glu Gly Ser Glu Val Ser Arg Val Thr Leu His Asn Glu Ser Tyr Ile
   355 360 365
   Glu Glu Leu Asp Ile Arg Ile Gly Asp Trp Val Leu Val His Lys Ala
   370 375 380
   Gly Gly Val Ile Pro Glu Val Leu Arg Val Leu Lys Glu Arg Arg Thr
   385 390 395 400
   Gly Glu Glu Arg Pro Ile Arg Trp Pro Glu Thr Cys Pro Glu Cys Gly
   405 410 415
   His Arg Leu Leu Lys Glu Gly Lys Val His Arg Cys Pro Asn Pro Leu
   420 425 430
   Cys Pro Ala Lys Arg Phe Glu Ala Ile Arg His Phe Ala Ser Arg Lys
   435 440 445
   Ala Met Asp Ile Gln Gly Leu Gly Glu Lys Leu Ile Glu Arg Leu Leu
   450 455 460
   Glu Lys Gly Leu Val Lys Asp Val Ala Asp Leu Tyr Arg Leu Arg Lys
   465 470 475 480
   Glu Asp Leu Val Gly Leu Glu Arg Met Gly Glu Lys Ser Ala Gln Asn
   485 490 495
   Leu Leu Arg Gln Ile Glu Glu Ser Lys Lys Arg Gly Leu Glu Arg Leu
   500 505 510
   Leu Tyr Ala Leu Gly Leu Pro Gly Val Gly Glu Val Leu Ala Arg Asn
   515 520 525
   Leu Ala Ala Arg Phe Gly Asn Met Asp Arg Leu Leu Glu Ala Ser Leu
   530 535 540
   Glu Glu Leu Leu Glu Val Glu Glu Val Gly Glu Leu Thr Ala Arg Ala
   545 550 555 560
   Ile Leu Glu Thr Leu Lys Asp Pro Ala Phe Arg Asp Leu Val Arg Arg
   565 570 575
   Leu Lys Glu Ala Gly Val Glu Met Glu Ala Lys Glu Lys Gly Gly Glu
   580 585 590
   Ala Leu Lys Gly Leu Thr Phe Val Ile Thr Gly Glu Leu Ser Arg Pro
   595 600 605
   Arg Glu Glu Val Lys Ala Leu Leu Arg Arg Leu Gly Ala Lys Val Thr
   610 615 620
   Asp Ser Val Ser Arg Lys Thr Ser Tyr Leu Val Val Gly Glu Asn Pro
   625 630 635 640
   Gly Ser Lys Leu Glu Lys Ala Arg Ala Leu Gly Val Pro Thr Leu Thr
   645 650 655
   Glu Glu Glu Leu Tyr Arg Leu Leu Glu Ala Arg Thr Gly Lys Lys Ala
   660 665 670
   Glu Glu Leu Val
   675
<210> 4
   <211> 22
   <212> PRT
   <213> Artificial Sequence
<220>
   <223> Description of Artificial Sequence: Synthetic peptide
<210> 5
   <211> 13
   <212> PRT
   <213> Artificial Sequence
<220>
   <223> Description of Artificial Sequence: Synthetic peptide
<400> 5
   Gly Ser Ser Gly Thr Ser Gly Gly Gly Ser Gly Gly Gly
   1               5                   10
<210> 6
   <211> 10
   <212> DNA
   <213> Unknown
<220>
   <223> Description of Unknown: Unknown natural target p50 sequence
<400> 6
   gggaattccc 10
<210> 7
   <211> 296
   <212> PRT
   <213> Artificial Sequence
<220>
   <223> Description of Artificial Sequence: Synthetic polypeptide
<400> 7
   Ser Pro Thr Ser Tyr Met Ser Pro Ser Leu Pro Ala Leu Asp Trp Gln
   1               5                   10                  15
   Leu Pro Ser His Ser Gly Pro Tyr Glu Leu Arg Ile Glu Val Gln Pro
               20                  25                  30
   Lys Ser His His Arg Ala His Tyr Glu Thr Glu Gly Ser Arg Gly Ala
           35                  40                  45
   Val Lys Ala Ser Ala Gly Gly His Pro Ile Val Gln Leu His Gly Tyr
       50                  55                  60
   Leu Glu Asn Glu Pro Leu Thr Leu Gln Leu Phe Ile Gly Thr Ala Asp
   65                  70                  75                  80
   Asp Arg Leu Leu Arg Pro His Ala Phe Tyr Gln Val His Arg Ile Thr
                   85                  90                  95
   Gly Lys Thr Val Ser Thr Thr Ser His Glu Ile Ile Leu Ser Asn Thr
               100                 105                 110
   Lys Val Leu Glu Ile Pro Leu Leu Pro Glu Asn Asn Met Arg Ala Ile
           115                 120                 125
   Ile Asp Cys Ala Gly Ile Leu Lys Leu Arg Asn Ser Asp Ile Glu Leu
       130                 135                 140
   Arg Lys Gly Glu Thr Asp Ile Gly Arg Lys Asn Thr Arg Val Arg Leu
   145                 150                 155                 160
   Val Phe Arg Val His Ile Pro Gln Pro Asn Gly Arg Thr Leu Ser Leu
                   165                 170                 175
   Gln Ala Ser Asn Leu Lys Ile Val Arg Met Asp Arg Thr Ala Gly Cys
               180                 185                 190
   Val Thr Gly Gly Glu Glu Ile Tyr Leu Leu Cys Asp Lys Val Gln Lys
           195                 200                 205
   Asp Asp Ile Gln Ile Arg Phe Tyr Glu Glu Glu Glu Asn Gly Gly Val
       210                 215                 220
   Trp Glu Gly Phe Gly Asp Phe Ser Pro Thr Asp Val His Arg Gln Phe
   225                 230                 235                 240
   Ala Ile Val Phe Lys Thr Pro Lys Tyr Lys Asp Ile Asn Ile Thr Lys
                   245                 250                 255
   Pro Ala Ser Val Phe Val Gln Leu Arg Arg Lys Ser Asp Leu Glu Thr
               260                 265                 270
   Ser Glu Pro Lys Pro Phe Leu Tyr Tyr Pro Glu Ile Lys Asp Lys Glu
           275                 280                 285
   Glu Val Gln Arg Lys Arg Gln Lys
       290                 295
<210> 8
   <211> 487
   <212> PRT
   <213> Enterobacteria phage T4
<400> 8
   Met Ile Leu Lys Ile Leu Asn Glu Ile Ala Ser Ile Gly Ser Thr Lys
   1               5                   10                  15
   Gln Lys Gln Ala Ile Leu Glu Lys Asn Lys Asp Asn Glu Leu Leu Lys
               20                  25                  30
   Arg Val Tyr Arg Leu Thr Tyr Ser Arg Gly Leu Gln Tyr Tyr Ile Lys
           35                  40                  45
   Lys Trp Pro Lys Pro Gly Ile Ala Thr Gln Ser Phe Gly Met Leu Thr
       50                  55                  60
   Leu Thr Asp Met Leu Asp Phe Ile Glu Phe Thr Leu Ala Thr Arg Lys
   65                  70                  75                  80
   Leu Thr Gly Asn Ala Ala Ile Glu Glu Leu Thr Gly Tyr Ile Thr Asp
                   85                  90                  95
   Gly Lys Lys Asp Asp Val Glu Val Leu Arg Arg Val Met Met Arg Asp
               100                 105                 110
   Leu Glu Cys Gly Ala Ser Val Ser Ile Ala Asn Lys Val Trp Pro Gly
           115                 120                 125
   Leu Ile Pro Glu Gln Pro Gln Met Leu Ala Ser Ser Tyr Asp Glu Lys
       130                 135                 140
   Gly Ile Asn Lys Asn Ile Lys Phe Pro Ala Phe Ala Gln Leu Lys Ala
   145                 150                 155                 160
   Asp Gly Ala Arg Cys Phe Ala Glu Val Arg Gly Asp Glu Leu Asp Asp
                   165                 170                 175
   Val Arg Leu Leu Ser Arg Ala Gly Asn Glu Tyr Leu Gly Leu Asp Leu
               180                 185                 190
   Leu Lys Glu Glu Leu Ile Lys Met Thr Ala Glu Ala Arg Gln Ile His
           195                 200                 205
   Pro Glu Gly Val Leu Ile Asp Gly Glu Leu Val Tyr His Glu Gln Val
       210                 215                 220
   Lys Lys Glu Pro Glu Gly Leu Asp Phe Leu Phe Asp Ala Tyr Pro Glu
   225                 230                 235                 240
   Asn Ser Lys Ala Lys Glu Phe Ala Glu Val Ala Glu Ser Arg Thr Ala
                   245                 250                 255
   Ser Asn Gly Ile Ala Asn Lys Ser Leu Lys Gly Thr Ile Ser Glu Lys
   260 265 270
   Glu Ala Gln Cys Met Lys Phe Gln Val Trp Asp Tyr Val Pro Leu Val
   275 280 285
   Glu Ile Tyr Ser Leu Pro Ala Phe Arg Leu Lys Tyr Asp Val Arg Phe
   290 295 300
   Ser Lys Leu Glu Gln Met Thr Ser Gly Tyr Asp Lys Val Ile Leu Ile
   305 310 315 320
   Glu Asn Gln Val Val Asn Asn Leu Asp Glu Ala Lys Val Ile Tyr Lys
   325 330 335
   Lys Tyr Ile Asp Gln Gly Leu Glu Gly Ile Ile Leu Lys Asn Ile Asp
   340 345 350
   Gly Leu Trp Glu Asn Ala Arg Ser Lys Asn Leu Tyr Lys Phe Lys Glu
   355 360 365
   Val Ile Asp Val Asp Leu Lys Ile Val Gly Ile Tyr Pro His Arg Lys
   370 375 380
   Asp Pro Thr Lys Ala Gly Gly Phe Ile Leu Glu Ser Glu Cys Gly Lys
   385 390 395 400
   Ile Lys Val Asn Ala Gly Ser Gly Leu Lys Asp Lys Ala Gly Val Lys
   405 410 415
   Ser His Glu Leu Asp Arg Thr Arg Ile Met Glu Asn Gln Asn Tyr Tyr
   420 425 430
   Ile Gly Lys Ile Leu Glu Cys Glu Cys Asn Gly Trp Leu Lys Ser Asp
   435 440 445
   Gly Arg Thr Asp Tyr Val Lys Leu Phe Leu Pro Ile Ala Ile Arg Leu
   450 455 460
   Arg Glu Asp Lys Thr Lys Ala Asn Thr Phe Glu Asp Val Phe Gly Asp
   465 470 475 480
   Phe His Glu Val Thr Gly Leu
   485
<210> 9
   <211> 559
   <212> PRT
   <213> Pyrococcus furiosus
<220>
   <223> Strain ST04
<400> 9
   Met Arg Tyr Leu Glu Leu Ala Gln Leu Tyr Gln Lys Leu Glu Lys Thr
   1               5                   10                  15
   Thr Met Lys Leu Ile Lys Thr Arg Leu Val Ala Asp Phe Leu Lys Lys
               20                  25                  30
   Val Pro Asp Asp His Leu Glu Phe Ile Pro Tyr Leu Ile Leu Gly Asp
           35                  40                  45
   Val Phe Pro Glu Trp Asp Glu Arg Glu Leu Gly Val Gly Glu Lys Leu
       50                  55                  60
   Leu Ile Lys Ala Val Ala Met Ala Thr Gly Ile Asp Ala Asn Glu Ile
   65                  70                  75                  80
   Glu Asn Ser Val Lys Asp Thr Gly Asp Leu Gly Glu Ser Ile Ala Leu
                   85                  90                  95
   Ala Val Lys Lys Arg Lys Gln Lys Ser Phe Phe Ser Gln Pro Leu Thr
               100                 105                 110
   Ile Lys Arg Val Tyr Gln Thr Leu Val Lys Val Ala Glu Thr Thr Gly
           115                 120                 125
   Glu Gly Ser Gln Glu Lys Lys Met Lys Tyr Leu Ala Asn Leu Phe Met
       130                 135                 140
   Asp Ala Glu Pro Ile Glu Ala Lys Tyr Ile Ala Arg Thr Val Leu Gly
   145                 150                 155                 160
   Thr Met Arg Thr Gly Val Ala Glu Gly Leu Leu Arg Asp Ala Ile Ala
                   165                 170                 175
   Leu Ala Phe His Val Lys Val Glu Leu Val Glu Arg Ala Tyr Met Leu
               180                 185                 190
   Thr Ser Asp Phe Gly Phe Val Ala Lys Val Ala Lys Leu Glu Gly Asn
           195                 200                 205
   Glu Gly Leu Ala Lys Val Gln Val Gln Ile Gly Lys Pro Ile Lys Pro
       210                 215                 220
   Met Leu Ala Gln Gln Ala Ala Asn Ile Lys Glu Ala Leu Leu Glu Met
   225 230 235 240
   Gly Gly Glu Ala Glu Phe Glu Ile Lys Tyr Asp Gly Ala Arg Val Gln
   245 250 255
   Val His Lys Asp Gly Asp Lys Ile Ile Val Tyr Ser Arg Arg Leu Glu
   260 265 270
   Asn Val Thr Arg Ala Ile Pro Glu Ile Val Glu Ala Leu Lys Gln Ser
   275 280 285
   Val Lys Pro Asn Lys Ala Ile Val Glu Gly Glu Leu Val Ala Ile Gly
   290 295 300
   Glu Asp Gly Arg Pro Leu Pro Phe Gln Tyr Val Leu Arg Arg Phe Arg
   305 310 315 320
   Arg Lys His Asn Ile Gln Glu Met Met Lys Lys Ile Pro Leu Glu Leu
   325 330 335
   Asn Leu Phe Asp Val Leu Tyr Val Asp Gly Glu Ser Met Ile Asp Val
   340 345 350
   Lys Phe Ile Asp Arg Arg Lys Lys Leu Glu Glu Ile Ile Glu Pro Asn
   355 360 365
   Gly Lys Ile Lys Val Ala Glu Asn Leu Ile Thr Lys Lys Val Glu Glu
   370 375 380
   Ala Glu Ala Phe Tyr Lys Lys Ala Leu Glu Met Gly His Glu Gly Leu
   385 390 395 400
   Met Ala Lys Arg Leu Asp Ala Thr Tyr Glu Pro Gly Asn Arg Gly Lys
   405 410 415
   Lys Trp Leu Lys Ile Lys Pro Thr Met Glu Asn Leu Asp Leu Val Ile
   420 425 430
   Ile Gly Ala Glu Trp Gly Glu Gly Arg Arg Ala His Leu Leu Gly Ser
   435 440 445
   Phe Ile Leu Gly Ala Tyr Asp Pro Glu Thr Gly Glu Phe Leu Glu Val
   450 455 460
   Gly Lys Val Gly Ser Gly Phe Thr Asp Glu Asp Leu Val Glu Phe Thr
   465 470 475 480
   Lys Met Leu Lys Pro Leu Ile Ile Lys Glu Glu Gly Lys Arg Val Trp
   485 490 495
   Ile Glu Pro Lys Ile Val Ile Glu Val Thr Tyr Gln Glu Ile Gln Lys
   500 505 510
   Ser Pro Lys Tyr Lys Ser Gly Phe Ala Leu Arg Phe Pro Arg Tyr Val
   515 520 525
   Ala Leu Arg Asp Asp Lys Gly Pro Glu Asp Ala Asp Thr Ile Glu Arg
   530 535 540
   Ile Ala Gln Leu Tyr Glu Leu Gln Glu Arg Met Lys Gly Lys Val
   545 550 555
<210> 10
   <211> 536
   <212> PRT
   <213> Pyrococcus furiosus
<220>
   <223> Strain ST04
<400> 10
   Met Arg Tyr Leu Glu Leu Ala Gln Leu Tyr Gln Lys Leu Glu Lys Thr
   1               5                   10                  15
   Thr Met Lys Leu Ile Lys Thr Arg Leu Val Ala Asp Phe Leu Lys Lys
               20                  25                  30
   Val Pro Asp Asp His Leu Glu Phe Ile Pro Tyr Leu Ile Leu Gly Asp
           35                  40                  45
   Val Phe Pro Glu Trp Asp Glu Arg Glu Leu Gly Val Gly Glu Lys Leu
       50                  55                  60
   Leu Ile Lys Ala Val Ala Met Ala Thr Gly Ile Asp Ala Asn Glu Ile
   65                  70                  75                  80
   Glu Asn Ser Val Lys Asp Thr Gly Asp Leu Gly Glu Ser Ile Ala Leu
                   85                  90                  95
   Ala Val Lys Lys Arg Lys Gln Lys Ser Phe Phe Ser Gln Pro Leu Thr
               100                 105                 110
   Ile Lys Arg Val Tyr Gln Thr Leu Val Lys Val Ala Glu Thr Thr Gly
           115                 120                 125
   Glu Gly Ser Gln Glu Lys Lys Met Lys Tyr Leu Ala Asn Leu Phe Met
       130                 135                 140
   Asp Ala Glu Pro Ile Glu Ala Lys Tyr Ile Ala Arg Thr Val Leu Gly
   145                 150                 155                 160
   Thr Met Arg Thr Gly Val Ala Glu Gly Leu Leu Arg Asp Ala Ile Ala
                   165                 170                 175
   Leu Ala Phe His Val Lys Val Glu Leu Val Glu Arg Ala Tyr Met Leu
               180                 185                 190
   Thr Ser Asp Phe Gly Phe Val Ala Lys Val Ala Lys Leu Glu Gly Asn
           195                 200                 205
   Glu Gly Leu Ala Lys Val Gln Val Gln Ile Gly Lys Pro Ile Lys Pro
       210                 215                 220
   Met Leu Ala Gln Gln Ala Ala Asn Ile Lys Glu Ala Leu Leu Glu Met
   225                 230                 235                 240
   Gly Gly Glu Ala Glu Phe Glu Ile Lys Tyr Asp Gly Ala Arg Val Gln
                   245                 250                 255
   Val His Lys Asp Gly Asp Lys Ile Ile Val Tyr Ser Arg Arg Leu Glu
               260                 265                 270
   Asn Val Thr Arg Ala Ile Pro Glu Ile Val Glu Ala Leu Lys Gln Ser
           275                 280                 285
   Val Lys Pro Asn Lys Ala Ile Val Glu Gly Glu Leu Val Ala Ile Gly
       290                 295                 300
   Glu Asp Gly Arg Pro Leu Pro Phe Gln Tyr Val Leu Arg Arg Phe Arg
   305                 310                 315                 320
   Arg Lys His Asn Ile Gln Glu Met Met Lys Lys Ile Pro Leu Glu Leu
                   325                 330                 335
   Asn Leu Phe Asp Val Leu Tyr Val Asp Gly Glu Ser Met Ile Asp Val
               340                 345                 350
   Lys Phe Ile Asp Arg Arg Lys Lys Leu Glu Glu Ile Ile Glu Pro Asn
           355                 360                 365
   Gly Lys Ile Lys Val Ala Glu Asn Leu Ile Thr Lys Lys Val Glu Glu
   370 375 380
   Ala Glu Ala Phe Tyr Lys Lys Ala Leu Glu Met Gly His Glu Gly Leu
   385 390 395 400
   Met Ala Lys Arg Leu Asp Ala Thr Tyr Glu Pro Gly Asn Arg Gly Lys
   405 410 415
   Lys Trp Leu Lys Ile Lys Pro Thr Met Glu Asn Leu Asp Leu Val Ile
   420 425 430
   Ile Gly Ala Glu Trp Gly Glu Gly Arg Arg Ala His Leu Leu Gly Ser
   435 440 445
   Phe Ile Leu Gly Ala Tyr Asp Pro Glu Thr Gly Glu Phe Leu Glu Val
   450 455 460
   Gly Lys Val Gly Ser Gly Phe Thr Asp Glu Asp Leu Val Glu Phe Thr
   465 470 475 480
   Lys Met Leu Lys Pro Leu Ile Ile Lys Glu Glu Gly Lys Arg Val Trp
   485 490 495
   Ile Glu Pro Lys Ile Val Ile Glu Val Thr Tyr Gln Glu Ile Gln Lys
   500 505 510
   Ser Pro Lys Tyr Lys Ser Gly Phe Ala Leu Arg Phe Pro Arg Tyr Val
   515 520 525
   Ala Leu Arg Asp Asp Lys Gly Pro
   530 535
<210> 11
   <211> 362
   <212> PRT
   <213> Artificial Sequence
<220>
   <223> Description of Artificial Sequence: Synthetic polypeptide
<400> 11
   Met Gly His His His His His His His His His His Ser Ser Gly His
   1               5                   10                  15
   Ile Glu Gly Arg Ala Ser Ala Asp Gly Pro Tyr Leu Gln Ile Leu Glu
               20                  25                  30
   Gln Pro Lys Gln Arg Gly Phe Arg Phe Arg Tyr Val Cys Glu Gly Pro
           35                  40                  45
   Ser His Gly Gly Leu Pro Gly Ala Ser Ser Glu Lys Asn Lys Lys Ser
       50                  55                  60
   Tyr Pro Gln Val Lys Ile Cys Asn Tyr Val Gly Pro Ala Lys Val Ile
   65                  70                  75                  80
   Val Gln Leu Val Thr Asn Gly Lys Asn Ile His Leu His Ala His Ser
                   85                  90                  95
   Leu Val Gly Lys His Cys Glu Asp Gly Ile Cys Thr Val Thr Ala Gly
               100                 105                 110
   Pro Lys Asp Met Val Val Gly Phe Ala Asn Leu Gly Ile Leu His Val
           115                 120                 125
   Thr Lys Lys Lys Val Phe Glu Thr Leu Glu Ala Arg Met Thr Glu Ala
       130                 135                 140
   Cys Ile Arg Gly Tyr Asn Pro Gly Leu Leu Val His Pro Asp Leu Ala
   145                 150                 155                 160
   Tyr Leu Gln Ala Glu Gly Gly Gly Asp Arg Gln Leu Gly Asp Arg Glu
                   165                 170                 175
   Lys Glu Leu Ile Arg Gln Ala Ala Leu Gln Gln Thr Lys Glu Met Asp
               180                 185                 190
   Leu Ser Val Val Arg Leu Met Phe Thr Ala Phe Leu Pro Asp Ser Thr
           195                 200                 205
   Gly Ser Phe Thr Arg Arg Leu Glu Pro Val Val Ser Asp Ala Ile Tyr
       210                 215                 220
   Asp Ser Lys Ala Pro Asn Ala Ser Asn Leu Lys Ile Val Arg Met Asp
   225                 230                 235                 240
   Arg Thr Ala Gly Cys Val Thr Gly Gly Glu Glu Ile Tyr Leu Leu Cys
                   245                 250                 255
   Asp Lys Val Gln Lys Asp Asp Ile Gln Ile Arg Phe Tyr Glu Glu Glu
               260                 265                 270
   Glu Asn Gly Gly Val Trp Glu Gly Phe Gly Asp Phe Ser Pro Thr Asp
           275                 280                 285
   Val His Arg Gln Phe Ala Ile Val Phe Lys Thr Pro Lys Tyr Lys Asp
   290 295 300
   Ile Asn Ile Thr Lys Pro Ala Ser Val Phe Val Gln Leu Arg Arg Lys
   305 310 315 320
   Ser Asp Leu Glu Thr Ser Glu Pro Lys Pro Phe Leu Tyr Tyr Pro Glu
   325 330 335
   Ile Lys Asp Lys Glu Glu Val Gln Arg Lys Arg Gln Lys Gly Ser Ser
   340 345 350
   Gly Thr Ser Gly Gly Gly Ser Gly Gly Gly
   355 360
<210> 12
   <211> 331
   <212> PRT
   <213> Artificial Sequence
<220>
   <223> Description of Artificial Sequence: Synthetic polypeptide
<400> 12
   Met Gly His His His His His His His His His His Ser Ser Gly His
   1               5                   10                  15
   Ile Glu Gly Arg Ala Ser Ser Pro Thr Ser Tyr Met Ser Pro Ser Leu
               20                  25                  30
   Pro Ala Leu Asp Trp Gln Leu Pro Ser His Ser Gly Pro Tyr Glu Leu
           35                  40                  45
   Arg Ile Glu Val Gln Pro Lys Ser His His Arg Ala His Tyr Glu Thr
       50                  55                  60
   Glu Gly Ser Arg Gly Ala Val Lys Ala Ser Ala Gly Gly His Pro Ile
   65                  70                  75                  80
   Val Gln Leu His Gly Tyr Leu Glu Asn Glu Pro Leu Thr Leu Gln Leu
                   85                  90                  95
   Phe Ile Gly Thr Ala Asp Asp Arg Leu Leu Arg Pro His Ala Phe Tyr
               100                 105                 110
   Gln Val His Arg Ile Thr Gly Lys Thr Val Ser Thr Thr Ser His Glu
           115                 120                 125
   Ile Ile Leu Ser Asn Thr Lys Val Leu Glu Ile Pro Leu Leu Pro Glu
       130                 135                 140
   Asn Asn Met Arg Ala Ile Ile Asp Cys Ala Gly Ile Leu Lys Leu Arg
   145                 150                 155                 160
   Asn Ser Asp Ile Glu Leu Arg Lys Gly Glu Thr Asp Ile Gly Arg Lys
                   165                 170                 175
   Asn Thr Arg Val Arg Leu Val Phe Arg Val His Ile Pro Gln Pro Asn
               180                 185                 190
   Gly Arg Thr Leu Ser Leu Gln Ala Ser Asn Leu Lys Ile Val Arg Met
           195                 200                 205
   Asp Arg Thr Ala Gly Cys Val Thr Gly Gly Glu Glu Ile Tyr Leu Leu
       210                 215                 220
   Cys Asp Lys Val Gln Lys Asp Asp Ile Gln Ile Arg Phe Tyr Glu Glu
   225                 230                 235                 240
   Glu Glu Asn Gly Gly Val Trp Glu Gly Phe Gly Asp Phe Ser Pro Thr
                   245                 250                 255
   Asp Val His Arg Gln Phe Ala Ile Val Phe Lys Thr Pro Lys Tyr Lys
               260                 265                 270
   Asp Ile Asn Ile Thr Lys Pro Ala Ser Val Phe Val Gln Leu Arg Arg
           275                 280                 285
   Lys Ser Asp Leu Glu Thr Ser Glu Pro Lys Pro Phe Leu Tyr Tyr Pro
       290                 295                 300
   Glu Ile Lys Asp Lys Glu Glu Val Gln Arg Lys Arg Gln Lys Gly Ser
   305                 310                 315                 320
   Ser Gly Thr Ser Gly Gly Gly Ser Gly Gly Gly
                   325                 330
<210> 13
   <211> 849
   <212> PRT
   <213> Artificial Sequence
<220>
   <223> Description of Artificial Sequence: Synthetic polypeptide
<400> 13
   Met Gly His His His His His His His His His His Ser Ser Gly His
   1               5                   10                  15
   Ile Glu Gly Arg Ala Ser Ala Asp Gly Pro Tyr Leu Gln Ile Leu Glu
               20                  25                  30
   Gln Pro Lys Gln Arg Gly Phe Arg Phe Arg Tyr Val Cys Glu Gly Pro
           35                  40                  45
   Ser His Gly Gly Leu Pro Gly Ala Ser Ser Glu Lys Asn Lys Lys Ser
       50                  55                  60
   Tyr Pro Gln Val Lys Ile Cys Asn Tyr Val Gly Pro Ala Lys Val Ile
   65                  70                  75                  80
   Val Gln Leu Val Thr Asn Gly Lys Asn Ile His Leu His Ala His Ser
                   85                  90                  95
   Leu Val Gly Lys His Cys Glu Asp Gly Ile Cys Thr Val Thr Ala Gly
               100                 105                 110
   Pro Lys Asp Met Val Val Gly Phe Ala Asn Leu Gly Ile Leu His Val
           115                 120                 125
   Thr Lys Lys Lys Val Phe Glu Thr Leu Glu Ala Arg Met Thr Glu Ala
       130                 135                 140
   Cys Ile Arg Gly Tyr Asn Pro Gly Leu Leu Val His Pro Asp Leu Ala
   145                 150                 155                 160
   Tyr Leu Gln Ala Glu Gly Gly Gly Asp Arg Gln Leu Gly Asp Arg Glu
                   165                 170                 175
   Lys Glu Leu Ile Arg Gln Ala Ala Leu Gln Gln Thr Lys Glu Met Asp
               180                 185                 190
   Leu Ser Val Val Arg Leu Met Phe Thr Ala Phe Leu Pro Asp Ser Thr
           195                 200                 205
   Gly Ser Phe Thr Arg Arg Leu Glu Pro Val Val Ser Asp Ala Ile Tyr
       210                 215                 220
   Asp Ser Lys Ala Pro Asn Ala Ser Asn Leu Lys Ile Val Arg Met Asp
   225                 230                 235                 240
   Arg Thr Ala Gly Cys Val Thr Gly Gly Glu Glu Ile Tyr Leu Leu Cys
                   245                 250                 255
   Asp Lys Val Gln Lys Asp Asp Ile Gln Ile Arg Phe Tyr Glu Glu Glu
               260                 265                 270
   Glu Asn Gly Gly Val Trp Glu Gly Phe Gly Asp Phe Ser Pro Thr Asp
   275 280 285
   Val His Arg Gln Phe Ala Ile Val Phe Lys Thr Pro Lys Tyr Lys Asp
   290 295 300
   Ile Asn Ile Thr Lys Pro Ala Ser Val Phe Val Gln Leu Arg Arg Lys
   305 310 315 320
   Ser Asp Leu Glu Thr Ser Glu Pro Lys Pro Phe Leu Tyr Tyr Pro Glu
   325 330 335
   Ile Lys Asp Lys Glu Glu Val Gln Arg Lys Arg Gln Lys Gly Ser Ser
   340 345 350
   Gly Thr Ser Gly Gly Gly Ser Gly Gly Gly Met Ile Leu Lys Ile Leu
   355 360 365
   Asn Glu Ile Ala Ser Ile Gly Ser Thr Lys Gln Lys Gln Ala Ile Leu
   370 375 380
   Glu Lys Asn Lys Asp Asn Glu Leu Leu Lys Arg Val Tyr Arg Leu Thr
   385 390 395 400
   Tyr Ser Arg Gly Leu Gln Tyr Tyr Ile Lys Lys Trp Pro Lys Pro Gly
   405 410 415
   Ile Ala Thr Gln Ser Phe Gly Met Leu Thr Leu Thr Asp Met Leu Asp
   420 425 430
   Phe Ile Glu Phe Thr Leu Ala Thr Arg Lys Leu Thr Gly Asn Ala Ala
   435 440 445
   Ile Glu Glu Leu Thr Gly Tyr Ile Thr Asp Gly Lys Lys Asp Asp Val
   450 455 460
   Glu Val Leu Arg Arg Val Met Met Arg Asp Leu Glu Cys Gly Ala Ser
   465 470 475 480
   Val Ser Ile Ala Asn Lys Val Trp Pro Gly Leu Ile Pro Glu Gln Pro
   485 490 495
   Gln Met Leu Ala Ser Ser Tyr Asp Glu Lys Gly Ile Asn Lys Asn Ile
   500 505 510
   Lys Phe Pro Ala Phe Ala Gln Leu Lys Ala Asp Gly Ala Arg Cys Phe
   515 520 525
   Ala Glu Val Arg Gly Asp Glu Leu Asp Asp Val Arg Leu Leu Ser Arg
   530 535 540
   Ala Gly Asn Glu Tyr Leu Gly Leu Asp Leu Leu Lys Glu Glu Leu Ile
   545 550 555 560
   Lys Met Thr Ala Glu Ala Arg Gln Ile His Pro Glu Gly Val Leu Ile
   565 570 575
   Asp Gly Glu Leu Val Tyr His Glu Gln Val Lys Lys Glu Pro Glu Gly
   580 585 590
   Leu Asp Phe Leu Phe Asp Ala Tyr Pro Glu Asn Ser Lys Ala Lys Glu
   595 600 605
   Phe Ala Glu Val Ala Glu Ser Arg Thr Ala Ser Asn Gly Ile Ala Asn
   610 615 620
   Lys Ser Leu Lys Gly Thr Ile Ser Glu Lys Glu Ala Gln Cys Met Lys
   625 630 635 640
   Phe Gln Val Trp Asp Tyr Val Pro Leu Val Glu Ile Tyr Ser Leu Pro
   645 650 655
   Ala Phe Arg Leu Lys Tyr Asp Val Arg Phe Ser Lys Leu Glu Gln Met
   660 665 670
   Thr Ser Gly Tyr Asp Lys Val Ile Leu Ile Glu Asn Gln Val Val Asn
   675 680 685
   Asn Leu Asp Glu Ala Lys Val Ile Tyr Lys Lys Tyr Ile Asp Gln Gly
   690 695 700
   Leu Glu Gly Ile Ile Leu Lys Asn Ile Asp Gly Leu Trp Glu Asn Ala
   705 710 715 720
   Arg Ser Lys Asn Leu Tyr Lys Phe Lys Glu Val Ile Asp Val Asp Leu
   725 730 735
   Lys Ile Val Gly Ile Tyr Pro His Arg Lys Asp Pro Thr Lys Ala Gly
   740 745 750
   Gly Phe Ile Leu Glu Ser Glu Cys Gly Lys Ile Lys Val Asn Ala Gly
   755 760 765
   Ser Gly Leu Lys Asp Lys Ala Gly Val Lys Ser His Glu Leu Asp Arg
   770 775 780
   Thr Arg Ile Met Glu Asn Gln Asn Tyr Tyr Ile Gly Lys Ile Leu Glu
   785 790 795 800
   Cys Glu Cys Asn Gly Trp Leu Lys Ser Asp Gly Arg Thr Asp Tyr Val
   805 810 815
   Lys Leu Phe Leu Pro Ile Ala Ile Arg Leu Arg Glu Asp Lys Thr Lys
   820 825 830
   Ala Asn Thr Phe Glu Asp Val Phe Gly Asp Phe His Glu Val Thr Gly
   835 840 845
   Leu
<210> 14
   <211> 921
   <212> PRT
   <213> Artificial Sequence
<220>
   <223> Description of Artificial Sequence: Synthetic polypeptide
<400> 14
   Met Gly His His His His His His His His His His Ser Ser Gly His
   1               5                   10                  15
   Ile Glu Gly Arg Ala Ser Ala Asp Gly Pro Tyr Leu Gln Ile Leu Glu
               20                  25                  30
   Gln Pro Lys Gln Arg Gly Phe Arg Phe Arg Tyr Val Cys Glu Gly Pro
           35                  40                  45
   Ser His Gly Gly Leu Pro Gly Ala Ser Ser Glu Lys Asn Lys Lys Ser
       50                  55                  60
   Tyr Pro Gln Val Lys Ile Cys Asn Tyr Val Gly Pro Ala Lys Val Ile
   65                  70                  75                  80
   Val Gln Leu Val Thr Asn Gly Lys Asn Ile His Leu His Ala His Ser
                   85                  90                  95
   Leu Val Gly Lys His Cys Glu Asp Gly Ile Cys Thr Val Thr Ala Gly
               100                 105                 110
   Pro Lys Asp Met Val Val Gly Phe Ala Asn Leu Gly Ile Leu His Val
   115 120 125
   Thr Lys Lys Lys Val Phe Glu Thr Leu Glu Ala Arg Met Thr Glu Ala
   130 135 140
   Cys Ile Arg Gly Tyr Asn Pro Gly Leu Leu Val His Pro Asp Leu Ala
   145 150 155 160
   Tyr Leu Gln Ala Glu Gly Gly Gly Asp Arg Gln Leu Gly Asp Arg Glu
   165 170 175
   Lys Glu Leu Ile Arg Gln Ala Ala Leu Gln Gln Thr Lys Glu Met Asp
   180 185 190
   Leu Ser Val Val Arg Leu Met Phe Thr Ala Phe Leu Pro Asp Ser Thr
   195 200 205
   Gly Ser Phe Thr Arg Arg Leu Glu Pro Val Val Ser Asp Ala Ile Tyr
   210 215 220
   Asp Ser Lys Ala Pro Asn Ala Ser Asn Leu Lys Ile Val Arg Met Asp
   225 230 235 240
   Arg Thr Ala Gly Cys Val Thr Gly Gly Glu Glu Ile Tyr Leu Leu Cys
   245 250 255
   Asp Lys Val Gln Lys Asp Asp Ile Gln Ile Arg Phe Tyr Glu Glu Glu
   260 265 270
   Glu Asn Gly Gly Val Trp Glu Gly Phe Gly Asp Phe Ser Pro Thr Asp
   275 280 285
   Val His Arg Gln Phe Ala Ile Val Phe Lys Thr Pro Lys Tyr Lys Asp
   290 295 300
   Ile Asn Ile Thr Lys Pro Ala Ser Val Phe Val Gln Leu Arg Arg Lys
   305 310 315 320
   Ser Asp Leu Glu Thr Ser Glu Pro Lys Pro Phe Leu Tyr Tyr Pro Glu
   325 330 335
   Ile Lys Asp Lys Glu Glu Val Gln Arg Lys Arg Gln Lys Gly Ser Ser
   340 345 350
   Gly Thr Ser Gly Gly Gly Ser Gly Gly Gly Met Arg Tyr Leu Glu Leu
   355 360 365
   Ala Gln Leu Tyr Gln Lys Leu Glu Lys Thr Thr Met Lys Leu Ile Lys
       370                 375                 380
   Thr Arg Leu Val Ala Asp Phe Leu Lys Lys Val Pro Asp Asp His Leu
   385                 390                 395                 400
   Glu Phe Ile Pro Tyr Leu Ile Leu Gly Asp Val Phe Pro Glu Trp Asp
                   405                 410                 415
   Glu Arg Glu Leu Gly Val Gly Glu Lys Leu Leu Ile Lys Ala Val Ala
               420                 425                 430
   Met Ala Thr Gly Ile Asp Ala Asn Glu Ile Glu Asn Ser Val Lys Asp
           435                 440                 445
   Thr Gly Asp Leu Gly Glu Ser Ile Ala Leu Ala Val Lys Lys Arg Lys
       450                 455                 460
   Gln Lys Ser Phe Phe Ser Gln Pro Leu Thr Ile Lys Arg Val Tyr Gln
   465                 470                 475                 480
   Thr Leu Val Lys Val Ala Glu Thr Thr Gly Glu Gly Ser Gln Glu Lys
                   485                 490                 495
   Lys Met Lys Tyr Leu Ala Asn Leu Phe Met Asp Ala Glu Pro Ile Glu
               500                 505                 510
   Ala Lys Tyr Ile Ala Arg Thr Val Leu Gly Thr Met Arg Thr Gly Val
           515                 520                 525
   Ala Glu Gly Leu Leu Arg Asp Ala Ile Ala Leu Ala Phe His Val Lys
       530                 535                 540
   Val Glu Leu Val Glu Arg Ala Tyr Met Leu Thr Ser Asp Phe Gly Phe
   545                 550                 555                 560
   Val Ala Lys Val Ala Lys Leu Glu Gly Asn Glu Gly Leu Ala Lys Val
                   565                 570                 575
   Gln Val Gln Ile Gly Lys Pro Ile Lys Pro Met Leu Ala Gln Gln Ala
               580                 585                 590
   Ala Asn Ile Lys Glu Ala Leu Leu Glu Met Gly Gly Glu Ala Glu Phe
           595                 600                 605
   Glu Ile Lys Tyr Asp Gly Ala Arg Val Gln Val His Lys Asp Gly Asp
       610                 615                 620
   Lys Ile Ile Val Tyr Ser Arg Arg Leu Glu Asn Val Thr Arg Ala Ile
   625 630 635 640
   Pro Glu Ile Val Glu Ala Leu Lys Gln Ser Val Lys Pro Asn Lys Ala
   645 650 655
   Ile Val Glu Gly Glu Leu Val Ala Ile Gly Glu Asp Gly Arg Pro Leu
   660 665 670
   Pro Phe Gln Tyr Val Leu Arg Arg Phe Arg Arg Lys His Asn Ile Gln
   675 680 685
   Glu Met Met Lys Lys Ile Pro Leu Glu Leu Asn Leu Phe Asp Val Leu
   690 695 700
   Tyr Val Asp Gly Glu Ser Met Ile Asp Val Lys Phe Ile Asp Arg Arg
   705 710 715 720
   Lys Lys Leu Glu Glu Ile Ile Glu Pro Asn Gly Lys Ile Lys Val Ala
   725 730 735
   Glu Asn Leu Ile Thr Lys Lys Val Glu Glu Ala Glu Ala Phe Tyr Lys
   740 745 750
   Lys Ala Leu Glu Met Gly His Glu Gly Leu Met Ala Lys Arg Leu Asp
   755 760 765
   Ala Thr Tyr Glu Pro Gly Asn Arg Gly Lys Lys Trp Leu Lys Ile Lys
   770 775 780
   Pro Thr Met Glu Asn Leu Asp Leu Val Ile Ile Gly Ala Glu Trp Gly
   785 790 795 800
   Glu Gly Arg Arg Ala His Leu Leu Gly Ser Phe Ile Leu Gly Ala Tyr
   805 810 815
   Asp Pro Glu Thr Gly Glu Phe Leu Glu Val Gly Lys Val Gly Ser Gly
   820 825 830
   Phe Thr Asp Glu Asp Leu Val Glu Phe Thr Lys Met Leu Lys Pro Leu
   835 840 845
   Ile Ile Lys Glu Glu Gly Lys Arg Val Trp Ile Glu Pro Lys Ile Val
   850 855 860
   Ile Glu Val Thr Tyr Gln Glu Ile Gln Lys Ser Pro Lys Tyr Lys Ser
   865 870 875 880
   Gly Phe Ala Leu Arg Phe Pro Arg Tyr Val Ala Leu Arg Asp Asp Lys
   885 890 895
   Gly Pro Glu Asp Ala Asp Thr Ile Glu Arg Ile Ala Gln Leu Tyr Glu
   900 905 910
   Leu Gln Glu Arg Met Lys Gly Lys Val
   915 920
<210> 15
   <211> 898
   <212> PRT
   <213> Artificial Sequence
<220>
   <223> Description of Artificial Sequence: Synthetic polypeptide
<400> 15
   Met Gly His His His His His His His His His His Ser Ser Gly His
   1 5 10 15
   Ile Glu Gly Arg Ala Ser Ala Asp Gly Pro Tyr Leu Gln Ile Leu Glu
   20 25 30
   Gln Pro Lys Gln Arg Gly Phe Arg Phe Arg Tyr Val Cys Glu Gly Pro
   35 40 45
   Ser His Gly Gly Leu Pro Gly Ala Ser Ser Glu Lys Asn Lys Lys Ser
   50 55 60
   Tyr Pro Gln Val Lys Ile Cys Asn Tyr Val Gly Pro Ala Lys Val Ile
   65 70 75 80
   Val Gln Leu Val Thr Asn Gly Lys Asn Ile His Leu His Ala His Ser
   85 90 95
   Leu Val Gly Lys His Cys Glu Asp Gly Ile Cys Thr Val Thr Ala Gly
   100 105 110
   Pro Lys Asp Met Val Val Gly Phe Ala Asn Leu Gly Ile Leu His Val
   115 120 125
   Thr Lys Lys Lys Val Phe Glu Thr Leu Glu Ala Arg Met Thr Glu Ala
   130 135 140
   Cys Ile Arg Gly Tyr Asn Pro Gly Leu Leu Val His Pro Asp Leu Ala
   145 150 155 160
   Tyr Leu Gln Ala Glu Gly Gly Gly Asp Arg Gln Leu Gly Asp Arg Glu
                   165                 170                 175
   Lys Glu Leu Ile Arg Gln Ala Ala Leu Gln Gln Thr Lys Glu Met Asp
               180                 185                 190
   Leu Ser Val Val Arg Leu Met Phe Thr Ala Phe Leu Pro Asp Ser Thr
           195                 200                 205
   Gly Ser Phe Thr Arg Arg Leu Glu Pro Val Val Ser Asp Ala Ile Tyr
       210                 215                 220
   Asp Ser Lys Ala Pro Asn Ala Ser Asn Leu Lys Ile Val Arg Met Asp
   225                 230                 235                 240
   Arg Thr Ala Gly Cys Val Thr Gly Gly Glu Glu Ile Tyr Leu Leu Cys
                   245                 250                 255
   Asp Lys Val Gln Lys Asp Asp Ile Gln Ile Arg Phe Tyr Glu Glu Glu
               260                 265                 270
   Glu Asn Gly Gly Val Trp Glu Gly Phe Gly Asp Phe Ser Pro Thr Asp
           275                 280                 285
   Val His Arg Gln Phe Ala Ile Val Phe Lys Thr Pro Lys Tyr Lys Asp
       290                 295                 300
   Ile Asn Ile Thr Lys Pro Ala Ser Val Phe Val Gln Leu Arg Arg Lys
   305                 310                 315                 320
   Ser Asp Leu Glu Thr Ser Glu Pro Lys Pro Phe Leu Tyr Tyr Pro Glu
                   325                 330                 335
   Ile Lys Asp Lys Glu Glu Val Gln Arg Lys Arg Gln Lys Gly Ser Ser
               340                 345                 350
   Gly Thr Ser Gly Gly Gly Ser Gly Gly Gly Met Arg Tyr Leu Glu Leu
           355                 360                 365
   Ala Gln Leu Tyr Gln Lys Leu Glu Lys Thr Thr Met Lys Leu Ile Lys
       370                 375                 380
   Thr Arg Leu Val Ala Asp Phe Leu Lys Lys Val Pro Asp Asp His Leu
   385                 390                 395                 400
   Glu Phe Ile Pro Tyr Leu Ile Leu Gly Asp Val Phe Pro Glu Trp Asp
                   405                 410                 415
   Glu Arg Glu Leu Gly Val Gly Glu Lys Leu Leu Ile Lys Ala Val Ala
   420 425 430
   Met Ala Thr Gly Ile Asp Ala Asn Glu Ile Glu Asn Ser Val Lys Asp
   435 440 445
   Thr Gly Asp Leu Gly Glu Ser Ile Ala Leu Ala Val Lys Lys Arg Lys
   450 455 460
   Gln Lys Ser Phe Phe Ser Gln Pro Leu Thr Ile Lys Arg Val Tyr Gln
   465 470 475 480
   Thr Leu Val Lys Val Ala Glu Thr Thr Gly Glu Gly Ser Gln Glu Lys
   485 490 495
   Lys Met Lys Tyr Leu Ala Asn Leu Phe Met Asp Ala Glu Pro Ile Glu
   500 505 510
   Ala Lys Tyr Ile Ala Arg Thr Val Leu Gly Thr Met Arg Thr Gly Val
   515 520 525
   Ala Glu Gly Leu Leu Arg Asp Ala Ile Ala Leu Ala Phe His Val Lys
   530 535 540
   Val Glu Leu Val Glu Arg Ala Tyr Met Leu Thr Ser Asp Phe Gly Phe
   545 550 555 560
   Val Ala Lys Val Ala Lys Leu Glu Gly Asn Glu Gly Leu Ala Lys Val
   565 570 575
   Gln Val Gln Ile Gly Lys Pro Ile Lys Pro Met Leu Ala Gln Gln Ala
   580 585 590
   Ala Asn Ile Lys Glu Ala Leu Leu Glu Met Gly Gly Glu Ala Glu Phe
   595 600 605
   Glu Ile Lys Tyr Asp Gly Ala Arg Val Gln Val His Lys Asp Gly Asp
   610 615 620
   Lys Ile Ile Val Tyr Ser Arg Arg Leu Glu Asn Val Thr Arg Ala Ile
   625 630 635 640
   Pro Glu Ile Val Glu Ala Leu Lys Gln Ser Val Lys Pro Asn Lys Ala
   645 650 655
   Ile Val Glu Gly Glu Leu Val Ala Ile Gly Glu Asp Gly Arg Pro Leu
               660                 665                 670
   Pro Phe Gln Tyr Val Leu Arg Arg Phe Arg Arg Lys His Asn Ile Gln
           675                 680                 685
   Glu Met Met Lys Lys Ile Pro Leu Glu Leu Asn Leu Phe Asp Val Leu
       690                 695                 700
   Tyr Val Asp Gly Glu Ser Met Ile Asp Val Lys Phe Ile Asp Arg Arg
   705                 710                 715                 720
   Lys Lys Leu Glu Glu Ile Ile Glu Pro Asn Gly Lys Ile Lys Val Ala
                   725                 730                 735
   Glu Asn Leu Ile Thr Lys Lys Val Glu Glu Ala Glu Ala Phe Tyr Lys
               740                 745                 750
   Lys Ala Leu Glu Met Gly His Glu Gly Leu Met Ala Lys Arg Leu Asp
           755                 760                 765
   Ala Thr Tyr Glu Pro Gly Asn Arg Gly Lys Lys Trp Leu Lys Ile Lys
       770                 775                 780
   Pro Thr Met Glu Asn Leu Asp Leu Val Ile Ile Gly Ala Glu Trp Gly
   785                 790                 795                 800
   Glu Gly Arg Arg Ala His Leu Leu Gly Ser Phe Ile Leu Gly Ala Tyr
                   805                 810                 815
   Asp Pro Glu Thr Gly Glu Phe Leu Glu Val Gly Lys Val Gly Ser Gly
               820                 825                 830
   Phe Thr Asp Glu Asp Leu Val Glu Phe Thr Lys Met Leu Lys Pro Leu
           835                 840                 845
   Ile Ile Lys Glu Glu Gly Lys Arg Val Trp Ile Glu Pro Lys Ile Val
       850                 855                 860
   Ile Glu Val Thr Tyr Gln Glu Ile Gln Lys Ser Pro Lys Tyr Lys Ser
   865                 870                 875                 880
   Gly Phe Ala Leu Arg Phe Pro Arg Tyr Val Ala Leu Arg Asp Asp Lys
                   885                 890                 895
   Gly Pro
<210> 16
   <211> 1007
   <212> PRT
   <213> Artificial Sequence
<220>
   <223> Description of Artificial Sequence: Synthetic polypeptide
<400> 16
   Met Gly His His His His His His His His His His Ser Ser Gly His
   1               5                   10                  15
   Ile Glu Gly Arg Ala Ser Ser Pro Thr Ser Tyr Met Ser Pro Ser Leu
               20                  25                  30
   Pro Ala Leu Asp Trp Gln Leu Pro Ser His Ser Gly Pro Tyr Glu Leu
           35                  40                  45
   Arg Ile Glu Val Gln Pro Lys Ser His His Arg Ala His Tyr Glu Thr
       50                  55                  60
   Glu Gly Ser Arg Gly Ala Val Lys Ala Ser Ala Gly Gly His Pro Ile
   65                  70                  75                  80
   Val Gln Leu His Gly Tyr Leu Glu Asn Glu Pro Leu Thr Leu Gln Leu
                   85                  90                  95
   Phe Ile Gly Thr Ala Asp Asp Arg Leu Leu Arg Pro His Ala Phe Tyr
               100                 105                 110
   Gln Val His Arg Ile Thr Gly Lys Thr Val Ser Thr Thr Ser His Glu
           115                 120                 125
   Ile Ile Leu Ser Asn Thr Lys Val Leu Glu Ile Pro Leu Leu Pro Glu
       130                 135                 140
   Asn Asn Met Arg Ala Ile Ile Asp Cys Ala Gly Ile Leu Lys Leu Arg
   145                 150                 155                 160
   Asn Ser Asp Ile Glu Leu Arg Lys Gly Glu Thr Asp Ile Gly Arg Lys
                   165                 170                 175
   Asn Thr Arg Val Arg Leu Val Phe Arg Val His Ile Pro Gln Pro Asn
               180                 185                 190
   Gly Arg Thr Leu Ser Leu Gln Ala Ser Asn Leu Lys Ile Val Arg Met
           195                 200                 205
   Asp Arg Thr Ala Gly Cys Val Thr Gly Gly Glu Glu Ile Tyr Leu Leu
       210                 215                 220
   Cys Asp Lys Val Gln Lys Asp Asp Ile Gln Ile Arg Phe Tyr Glu Glu
   225                 230                 235                 240
   Glu Glu Asn Gly Gly Val Trp Glu Gly Phe Gly Asp Phe Ser Pro Thr
                   245                 250                 255
   Asp Val His Arg Gln Phe Ala Ile Val Phe Lys Thr Pro Lys Tyr Lys
               260                 265                 270
   Asp Ile Asn Ile Thr Lys Pro Ala Ser Val Phe Val Gln Leu Arg Arg
           275                 280                 285
   Lys Ser Asp Leu Glu Thr Ser Glu Pro Lys Pro Phe Leu Tyr Tyr Pro
       290                 295                 300
   Glu Ile Lys Asp Lys Glu Glu Val Gln Arg Lys Arg Gln Lys Gly Ser
   305                 310                 315                 320
   Ser Gly Thr Ser Gly Gly Gly Ser Gly Gly Gly Met Thr Leu Glu Glu
                   325                 330                 335
   Ala Arg Lys Arg Val Asn Glu Leu Arg Asp Leu Ile Arg Tyr His Asn
               340                 345                 350
   Tyr Arg Tyr Tyr Val Leu Ala Asp Pro Glu Ile Ser Asp Ala Glu Tyr
           355                 360                 365
   Asp Arg Leu Leu Arg Glu Leu Lys Glu Leu Glu Glu Arg Phe Pro Glu
       370                 375                 380
   Leu Lys Ser Pro Asp Ser Pro Thr Leu Gln Val Gly Ala Arg Pro Leu
   385                 390                 395                 400
   Glu Ala Thr Phe Arg Pro Val Arg His Pro Thr Arg Met Tyr Ser Leu
                   405                 410                 415
   Asp Asn Ala Phe Asn Leu Asp Glu Leu Lys Ala Phe Glu Glu Arg Ile
               420                 425                 430
   Glu Arg Ala Leu Gly Arg Lys Gly Pro Phe Ala Tyr Thr Val Glu His
           435                 440                 445
   Lys Val Asp Gly Leu Ser Val Asn Leu Tyr Tyr Glu Glu Gly Val Leu
       450                 455                 460
   Val Tyr Gly Ala Thr Arg Gly Asp Gly Glu Val Gly Glu Glu Val Thr
   465 470 475 480
   Gln Asn Leu Leu Thr Ile Pro Thr Ile Pro Arg Arg Leu Lys Gly Val
   485 490 495
   Pro Glu Arg Leu Glu Val Arg Gly Glu Val Tyr Met Pro Ile Glu Ala
   500 505 510
   Phe Leu Arg Leu Asn Glu Glu Leu Glu Glu Arg Gly Glu Arg Ile Phe
   515 520 525
   Lys Asn Pro Arg Asn Ala Ala Ala Gly Ser Leu Arg Gln Lys Asp Pro
   530 535 540
   Arg Ile Thr Ala Lys Arg Gly Leu Arg Ala Thr Phe Tyr Ala Leu Gly
   545 550 555 560
   Leu Gly Leu Glu Glu Val Glu Arg Glu Gly Val Ala Thr Gln Phe Ala
   565 570 575
   Leu Leu His Trp Leu Lys Glu Lys Gly Phe Pro Val Glu His Gly Tyr
   580 585 590
   Ala Arg Ala Val Gly Ala Glu Gly Val Glu Ala Val Tyr Gln Asp Trp
   595 600 605
   Leu Lys Lys Arg Arg Ala Leu Pro Phe Glu Ala Asp Gly Val Val Val
   610 615 620
   Lys Leu Asp Glu Leu Ala Leu Trp Arg Glu Leu Gly Tyr Thr Ala Arg
   625 630 635 640
   Ala Pro Arg Phe Ala Ile Ala Tyr Lys Phe Pro Ala Glu Glu Lys Glu
   645 650 655
   Thr Arg Leu Leu Asp Val Val Phe Gln Val Gly Arg Thr Gly Arg Val
   660 665 670
   Thr Pro Val Gly Ile Leu Glu Pro Val Phe Leu Glu Gly Ser Glu Val
   675 680 685
   Ser Arg Val Thr Leu His Asn Glu Ser Tyr Ile Glu Glu Leu Asp Ile
   690 695 700
   Arg Ile Gly Asp Trp Val Leu Val His Lys Ala Gly Gly Val Ile Pro
   705 710 715 720
   Glu Val Leu Arg Val Leu Lys Glu Arg Arg Thr Gly Glu Glu Arg Pro
                   725                 730                 735
   Ile Arg Trp Pro Glu Thr Cys Pro Glu Cys Gly His Arg Leu Leu Lys
               740                 745                 750
   Glu Gly Lys Val His Arg Cys Pro Asn Pro Leu Cys Pro Ala Lys Arg
           755                 760                 765
   Phe Glu Ala Ile Arg His Phe Ala Ser Arg Lys Ala Met Asp Ile Gln
       770                 775                 780
   Gly Leu Gly Glu Lys Leu Ile Glu Arg Leu Leu Glu Lys Gly Leu Val
   785                 790                 795                 800
   Lys Asp Val Ala Asp Leu Tyr Arg Leu Arg Lys Glu Asp Leu Val Gly
                   805                 810                 815
   Leu Glu Arg Met Gly Glu Lys Ser Ala Gln Asn Leu Leu Arg Gln Ile
               820                 825                 830
   Glu Glu Ser Lys Lys Arg Gly Leu Glu Arg Leu Leu Tyr Ala Leu Gly
           835                 840                 845
   Leu Pro Gly Val Gly Glu Val Leu Ala Arg Asn Leu Ala Ala Arg Phe
       850                 855                 860
   Gly Asn Met Asp Arg Leu Leu Glu Ala Ser Leu Glu Glu Leu Leu Glu
   865                 870                 875                 880
   Val Glu Glu Val Gly Glu Leu Thr Ala Arg Ala Ile Leu Glu Thr Leu
                   885                 890                 895
   Lys Asp Pro Ala Phe Arg Asp Leu Val Arg Arg Leu Lys Glu Ala Gly
               900                 905                 910
   Val Glu Met Glu Ala Lys Glu Lys Gly Gly Glu Ala Leu Lys Gly Leu
           915                 920                 925
   Thr Phe Val Ile Thr Gly Glu Leu Ser Arg Pro Arg Glu Glu Val Lys
       930                 935                 940
   Ala Leu Leu Arg Arg Leu Gly Ala Lys Val Thr Asp Ser Val Ser Arg
   945                 950                 955                 960
   Lys Thr Ser Tyr Leu Val Val Gly Glu Asn Pro Gly Ser Lys Leu Glu
                   965                 970                 975
   Lys Ala Arg Ala Leu Gly Val Pro Thr Leu Thr Glu Glu Glu Leu Tyr
   980 985 990
   Arg Leu Leu Glu Ala Arg Thr Gly Lys Lys Ala Glu Glu Leu Val
   995 1000 1005
<210> 17
   <211> 818
   <212> PRT
   <213> Artificial Sequence
<220>
   <223> Description of Artificial Sequence: Synthetic polypeptide
<400> 17
   Met Gly His His His His His His His His His His Ser Ser Gly His
   1 5 10 15
   Ile Glu Gly Arg Ala Ser Ser Pro Thr Ser Tyr Met Ser Pro Ser Leu
   20 25 30
   Pro Ala Leu Asp Trp Gln Leu Pro Ser His Ser Gly Pro Tyr Glu Leu
   35 40 45
   Arg Ile Glu Val Gln Pro Lys Ser His His Arg Ala His Tyr Glu Thr
   50 55 60
   Glu Gly Ser Arg Gly Ala Val Lys Ala Ser Ala Gly Gly His Pro Ile
   65 70 75 80
   Val Gln Leu His Gly Tyr Leu Glu Asn Glu Pro Leu Thr Leu Gln Leu
   85 90 95
   Phe Ile Gly Thr Ala Asp Asp Arg Leu Leu Arg Pro His Ala Phe Tyr
   100 105 110
   Gln Val His Arg Ile Thr Gly Lys Thr Val Ser Thr Thr Ser His Glu
   115 120 125
   Ile Ile Leu Ser Asn Thr Lys Val Leu Glu Ile Pro Leu Leu Pro Glu
   130 135 140
   Asn Asn Met Arg Ala Ile Ile Asp Cys Ala Gly Ile Leu Lys Leu Arg
   145 150 155 160
   Asn Ser Asp Ile Glu Leu Arg Lys Gly Glu Thr Asp Ile Gly Arg Lys
   165 170 175
   Asn Thr Arg Val Arg Leu Val Phe Arg Val His Ile Pro Gln Pro Asn
               180                 185                 190
   Gly Arg Thr Leu Ser Leu Gln Ala Ser Asn Leu Lys Ile Val Arg Met
           195                 200                 205
   Asp Arg Thr Ala Gly Cys Val Thr Gly Gly Glu Glu Ile Tyr Leu Leu
       210                 215                 220
   Cys Asp Lys Val Gln Lys Asp Asp Ile Gln Ile Arg Phe Tyr Glu Glu
   225                 230                 235                 240
   Glu Glu Asn Gly Gly Val Trp Glu Gly Phe Gly Asp Phe Ser Pro Thr
                   245                 250                 255
   Asp Val His Arg Gln Phe Ala Ile Val Phe Lys Thr Pro Lys Tyr Lys
               260                 265                 270
   Asp Ile Asn Ile Thr Lys Pro Ala Ser Val Phe Val Gln Leu Arg Arg
           275                 280                 285
   Lys Ser Asp Leu Glu Thr Ser Glu Pro Lys Pro Phe Leu Tyr Tyr Pro
       290                 295                 300
   Glu Ile Lys Asp Lys Glu Glu Val Gln Arg Lys Arg Gln Lys Gly Ser
   305                 310                 315                 320
   Ser Gly Thr Ser Gly Gly Gly Ser Gly Gly Gly Met Ile Leu Lys Ile
                   325                 330                 335
   Leu Asn Glu Ile Ala Ser Ile Gly Ser Thr Lys Gln Lys Gln Ala Ile
               340                 345                 350
   Leu Glu Lys Asn Lys Asp Asn Glu Leu Leu Lys Arg Val Tyr Arg Leu
           355                 360                 365
   Thr Tyr Ser Arg Gly Leu Gln Tyr Tyr Ile Lys Lys Trp Pro Lys Pro
       370                 375                 380
   Gly Ile Ala Thr Gln Ser Phe Gly Met Leu Thr Leu Thr Asp Met Leu
   385                 390                 395                 400
   Asp Phe Ile Glu Phe Thr Leu Ala Thr Arg Lys Leu Thr Gly Asn Ala
                   405                 410                 415
   Ala Ile Glu Glu Leu Thr Gly Tyr Ile Thr Asp Gly Lys Lys Asp Asp
               420                 425                 430
   Val Glu Val Leu Arg Arg Val Met Met Arg Asp Leu Glu Cys Gly Ala
   435 440 445
   Ser Val Ser Ile Ala Asn Lys Val Trp Pro Gly Leu Ile Pro Glu Gln
   450 455 460
   Pro Gln Met Leu Ala Ser Ser Tyr Asp Glu Lys Gly Ile Asn Lys Asn
   465 470 475 480
   Ile Lys Phe Pro Ala Phe Ala Gln Leu Lys Ala Asp Gly Ala Arg Cys
   485 490 495
   Phe Ala Glu Val Arg Gly Asp Glu Leu Asp Asp Val Arg Leu Leu Ser
   500 505 510
   Arg Ala Gly Asn Glu Tyr Leu Gly Leu Asp Leu Leu Lys Glu Glu Leu
   515 520 525
   Ile Lys Met Thr Ala Glu Ala Arg Gln Ile His Pro Glu Gly Val Leu
   530 535 540
   Ile Asp Gly Glu Leu Val Tyr His Glu Gln Val Lys Lys Glu Pro Glu
   545 550 555 560
   Gly Leu Asp Phe Leu Phe Asp Ala Tyr Pro Glu Asn Ser Lys Ala Lys
   565 570 575
   Glu Phe Ala Glu Val Ala Glu Ser Arg Thr Ala Ser Asn Gly Ile Ala
   580 585 590
   Asn Lys Ser Leu Lys Gly Thr Ile Ser Glu Lys Glu Ala Gln Cys Met
   595 600 605
   Lys Phe Gln Val Trp Asp Tyr Val Pro Leu Val Glu Ile Tyr Ser Leu
   610 615 620
   Pro Ala Phe Arg Leu Lys Tyr Asp Val Arg Phe Ser Lys Leu Glu Gln
   625 630 635 640
   Met Thr Ser Gly Tyr Asp Lys Val Ile Leu Ile Glu Asn Gln Val Val
   645 650 655
   Asn Asn Leu Asp Glu Ala Lys Val Ile Tyr Lys Lys Tyr Ile Asp Gln
   660 665 670
   Gly Leu Glu Gly Ile Ile Leu Lys Asn Ile Asp Gly Leu Trp Glu Asn
   675 680 685
   Ala Arg Ser Lys Asn Leu Tyr Lys Phe Lys Glu Val Ile Asp Val Asp
   690 695 700
   Leu Lys Ile Val Gly Ile Tyr Pro His Arg Lys Asp Pro Thr Lys Ala
   705 710 715 720
   Gly Gly Phe Ile Leu Glu Ser Glu Cys Gly Lys Ile Lys Val Asn Ala
   725 730 735
   Gly Ser Gly Leu Lys Asp Lys Ala Gly Val Lys Ser His Glu Leu Asp
   740 745 750
   Arg Thr Arg Ile Met Glu Asn Gln Asn Tyr Tyr Ile Gly Lys Ile Leu
   755 760 765
   Glu Cys Glu Cys Asn Gly Trp Leu Lys Ser Asp Gly Arg Thr Asp Tyr
   770 775 780
   Val Lys Leu Phe Leu Pro Ile Ala Ile Arg Leu Arg Glu Asp Lys Thr
   785 790 795 800
   Lys Ala Asn Thr Phe Glu Asp Val Phe Gly Asp Phe His Glu Val Thr
   805 810 815
   Gly Leu
<210> 18
   <211> 890
   <212> PRT
   <213> Artificial Sequence
<220>
   <223> Description of Artificial Sequence: Synthetic polypeptide
<400> 18
   Met Gly His His His His His His His His His His Ser Ser Gly His
   1               5                   10                  15
   Ile Glu Gly Arg Ala Ser Ser Pro Thr Ser Tyr Met Ser Pro Ser Leu
               20                  25                  30
   Pro Ala Leu Asp Trp Gln Leu Pro Ser His Ser Gly Pro Tyr Glu Leu
           35                  40                  45
   Arg Ile Glu Val Gln Pro Lys Ser His His Arg Ala His Tyr Glu Thr
       50                  55                  60
   Glu Gly Ser Arg Gly Ala Val Lys Ala Ser Ala Gly Gly His Pro Ile
   65                  70                  75                  80
   Val Gln Leu His Gly Tyr Leu Glu Asn Glu Pro Leu Thr Leu Gln Leu
                   85                  90                  95
   Phe Ile Gly Thr Ala Asp Asp Arg Leu Leu Arg Pro His Ala Phe Tyr
               100                 105                 110
   Gln Val His Arg Ile Thr Gly Lys Thr Val Ser Thr Thr Ser His Glu
           115                 120                 125
   Ile Ile Leu Ser Asn Thr Lys Val Leu Glu Ile Pro Leu Leu Pro Glu
       130                 135                 140
   Asn Asn Met Arg Ala Ile Ile Asp Cys Ala Gly Ile Leu Lys Leu Arg
   145                 150                 155                 160
   Asn Ser Asp Ile Glu Leu Arg Lys Gly Glu Thr Asp Ile Gly Arg Lys
                   165                 170                 175
   Asn Thr Arg Val Arg Leu Val Phe Arg Val His Ile Pro Gln Pro Asn
               180                 185                 190
   Gly Arg Thr Leu Ser Leu Gln Ala Ser Asn Leu Lys Ile Val Arg Met
           195                 200                 205
   Asp Arg Thr Ala Gly Cys Val Thr Gly Gly Glu Glu Ile Tyr Leu Leu
       210                 215                 220
   Cys Asp Lys Val Gln Lys Asp Asp Ile Gln Ile Arg Phe Tyr Glu Glu
   225                 230                 235                 240
   Glu Glu Asn Gly Gly Val Trp Glu Gly Phe Gly Asp Phe Ser Pro Thr
                   245                 250                 255
   Asp Val His Arg Gln Phe Ala Ile Val Phe Lys Thr Pro Lys Tyr Lys
               260                 265                 270
   Asp Ile Asn Ile Thr Lys Pro Ala Ser Val Phe Val Gln Leu Arg Arg
           275                 280                 285
   Lys Ser Asp Leu Glu Thr Ser Glu Pro Lys Pro Phe Leu Tyr Tyr Pro
       290                 295                 300
   Glu Ile Lys Asp Lys Glu Glu Val Gln Arg Lys Arg Gln Lys Gly Ser
   305 310 315 320
   Ser Gly Thr Ser Gly Gly Gly Ser Gly Gly Gly Met Arg Tyr Leu Glu
   325 330 335
   Leu Ala Gln Leu Tyr Gln Lys Leu Glu Lys Thr Thr Met Lys Leu Ile
   340 345 350
   Lys Thr Arg Leu Val Ala Asp Phe Leu Lys Lys Val Pro Asp Asp His
   355 360 365
   Leu Glu Phe Ile Pro Tyr Leu Ile Leu Gly Asp Val Phe Pro Glu Trp
   370 375 380
   Asp Glu Arg Glu Leu Gly Val Gly Glu Lys Leu Leu Ile Lys Ala Val
   385 390 395 400
   Ala Met Ala Thr Gly Ile Asp Ala Asn Glu Ile Glu Asn Ser Val Lys
   405 410 415
   Asp Thr Gly Asp Leu Gly Glu Ser Ile Ala Leu Ala Val Lys Lys Arg
   420 425 430
   Lys Gln Lys Ser Phe Phe Ser Gln Pro Leu Thr Ile Lys Arg Val Tyr
   435 440 445
   Gln Thr Leu Val Lys Val Ala Glu Thr Thr Gly Glu Gly Ser Gln Glu
   450 455 460
   Lys Lys Met Lys Tyr Leu Ala Asn Leu Phe Met Asp Ala Glu Pro Ile
   465 470 475 480
   Glu Ala Lys Tyr Ile Ala Arg Thr Val Leu Gly Thr Met Arg Thr Gly
   485 490 495
   Val Ala Glu Gly Leu Leu Arg Asp Ala Ile Ala Leu Ala Phe His Val
   500 505 510
   Lys Val Glu Leu Val Glu Arg Ala Tyr Met Leu Thr Ser Asp Phe Gly
   515 520 525
   Phe Val Ala Lys Val Ala Lys Leu Glu Gly Asn Glu Gly Leu Ala Lys
   530 535 540
   Val Gln Val Gln Ile Gly Lys Pro Ile Lys Pro Met Leu Ala Gln Gln
   545 550 555 560
   Ala Ala Asn Ile Lys Glu Ala Leu Leu Glu Met Gly Gly Glu Ala Glu
                   565                 570                 575
   Phe Glu Ile Lys Tyr Asp Gly Ala Arg Val Gln Val His Lys Asp Gly
               580                 585                 590
   Asp Lys Ile Ile Val Tyr Ser Arg Arg Leu Glu Asn Val Thr Arg Ala
           595                 600                 605
   Ile Pro Glu Ile Val Glu Ala Leu Lys Gln Ser Val Lys Pro Asn Lys
       610                 615                 620
   Ala Ile Val Glu Gly Glu Leu Val Ala Ile Gly Glu Asp Gly Arg Pro
   625                 630                 635                 640
   Leu Pro Phe Gln Tyr Val Leu Arg Arg Phe Arg Arg Lys His Asn Ile
                   645                 650                 655
   Gln Glu Met Met Lys Lys Ile Pro Leu Glu Leu Asn Leu Phe Asp Val
               660                 665                 670
   Leu Tyr Val Asp Gly Glu Ser Met Ile Asp Val Lys Phe Ile Asp Arg
           675                 680                 685
   Arg Lys Lys Leu Glu Glu Ile Ile Glu Pro Asn Gly Lys Ile Lys Val
       690                 695                 700
   Ala Glu Asn Leu Ile Thr Lys Lys Val Glu Glu Ala Glu Ala Phe Tyr
   705                 710                 715                 720
   Lys Lys Ala Leu Glu Met Gly His Glu Gly Leu Met Ala Lys Arg Leu
                   725                 730                 735
   Asp Ala Thr Tyr Glu Pro Gly Asn Arg Gly Lys Lys Trp Leu Lys Ile
               740                 745                 750
   Lys Pro Thr Met Glu Asn Leu Asp Leu Val Ile Ile Gly Ala Glu Trp
           755                 760                 765
   Gly Glu Gly Arg Arg Ala His Leu Leu Gly Ser Phe Ile Leu Gly Ala
       770                 775                 780
   Tyr Asp Pro Glu Thr Gly Glu Phe Leu Glu Val Gly Lys Val Gly Ser
   785                 790                 795                 800
   Gly Phe Thr Asp Glu Asp Leu Val Glu Phe Thr Lys Met Leu Lys Pro
                   805                 810                 815
   Leu Ile Ile Lys Glu Glu Gly Lys Arg Val Trp Ile Glu Pro Lys Ile
               820                 825                 830
   Val Ile Glu Val Thr Tyr Gln Glu Ile Gln Lys Ser Pro Lys Tyr Lys
           835                 840                 845
   Ser Gly Phe Ala Leu Arg Phe Pro Arg Tyr Val Ala Leu Arg Asp Asp
       850                 855                 860
   Lys Gly Pro Glu Asp Ala Asp Thr Ile Glu Arg Ile Ala Gln Leu Tyr
   865                 870                 875                 880
   Glu Leu Gln Glu Arg Met Lys Gly Lys Val
                   885                 890
<210> 19
   <211> 867
   <212> PRT
   <213> Artificial Sequence
<220>
   <223> Description of Artificial Sequence: Synthetic polypeptide
<400> 19
   Met Gly His His His His His His His His His His Ser Ser Gly His
   1               5                   10                  15
   Ile Glu Gly Arg Ala Ser Ser Pro Thr Ser Tyr Met Ser Pro Ser Leu
               20                  25                  30
   Pro Ala Leu Asp Trp Gln Leu Pro Ser His Ser Gly Pro Tyr Glu Leu
           35                  40                  45
   Arg Ile Glu Val Gln Pro Lys Ser His His Arg Ala His Tyr Glu Thr
       50                  55                  60
   Glu Gly Ser Arg Gly Ala Val Lys Ala Ser Ala Gly Gly His Pro Ile
   65                  70                  75                  80
   Val Gln Leu His Gly Tyr Leu Glu Asn Glu Pro Leu Thr Leu Gln Leu
                   85                  90                  95
   Phe Ile Gly Thr Ala Asp Asp Arg Leu Leu Arg Pro His Ala Phe Tyr
               100                 105                 110
   Gln Val His Arg Ile Thr Gly Lys Thr Val Ser Thr Thr Ser His Glu
           115                 120                 125
   Ile Ile Leu Ser Asn Thr Lys Val Leu Glu Ile Pro Leu Leu Pro Glu
       130                 135                 140
   Asn Asn Met Arg Ala Ile Ile Asp Cys Ala Gly Ile Leu Lys Leu Arg
   145                 150                 155                 160
   Asn Ser Asp Ile Glu Leu Arg Lys Gly Glu Thr Asp Ile Gly Arg Lys
                   165                 170                 175
   Asn Thr Arg Val Arg Leu Val Phe Arg Val His Ile Pro Gln Pro Asn
               180                 185                 190
   Gly Arg Thr Leu Ser Leu Gln Ala Ser Asn Leu Lys Ile Val Arg Met
           195                 200                 205
   Asp Arg Thr Ala Gly Cys Val Thr Gly Gly Glu Glu Ile Tyr Leu Leu
       210                 215                 220
   Cys Asp Lys Val Gln Lys Asp Asp Ile Gln Ile Arg Phe Tyr Glu Glu
   225                 230                 235                 240
   Glu Glu Asn Gly Gly Val Trp Glu Gly Phe Gly Asp Phe Ser Pro Thr
                   245                 250                 255
   Asp Val His Arg Gln Phe Ala Ile Val Phe Lys Thr Pro Lys Tyr Lys
               260                 265                 270
   Asp Ile Asn Ile Thr Lys Pro Ala Ser Val Phe Val Gln Leu Arg Arg
           275                 280                 285
   Lys Ser Asp Leu Glu Thr Ser Glu Pro Lys Pro Phe Leu Tyr Tyr Pro
       290                 295                 300
   Glu Ile Lys Asp Lys Glu Glu Val Gln Arg Lys Arg Gln Lys Gly Ser
   305                 310                 315                 320
   Ser Gly Thr Ser Gly Gly Gly Ser Gly Gly Gly Met Arg Tyr Leu Glu
                   325                 330                 335
   Leu Ala Gln Leu Tyr Gln Lys Leu Glu Lys Thr Thr Met Lys Leu Ile
               340                 345                 350
   Lys Thr Arg Leu Val Ala Asp Phe Leu Lys Lys Val Pro Asp Asp His
           355                 360                 365
   Leu Glu Phe Ile Pro Tyr Leu Ile Leu Gly Asp Val Phe Pro Glu Trp
       370                 375                 380
   Asp Glu Arg Glu Leu Gly Val Gly Glu Lys Leu Leu Ile Lys Ala Val
   385 390 395 400
   Ala Met Ala Thr Gly Ile Asp Ala Asn Glu Ile Glu Asn Ser Val Lys
   405 410 415
   Asp Thr Gly Asp Leu Gly Glu Ser Ile Ala Leu Ala Val Lys Lys Arg
   420 425 430
   Lys Gln Lys Ser Phe Phe Ser Gln Pro Leu Thr Ile Lys Arg Val Tyr
   435 440 445
   Gln Thr Leu Val Lys Val Ala Glu Thr Thr Gly Glu Gly Ser Gln Glu
   450 455 460
   Lys Lys Met Lys Tyr Leu Ala Asn Leu Phe Met Asp Ala Glu Pro Ile
   465 470 475 480
   Glu Ala Lys Tyr Ile Ala Arg Thr Val Leu Gly Thr Met Arg Thr Gly
   485 490 495
   Val Ala Glu Gly Leu Leu Arg Asp Ala Ile Ala Leu Ala Phe His Val
   500 505 510
   Lys Val Glu Leu Val Glu Arg Ala Tyr Met Leu Thr Ser Asp Phe Gly
   515 520 525
   Phe Val Ala Lys Val Ala Lys Leu Glu Gly Asn Glu Gly Leu Ala Lys
   530 535 540
   Val Gln Val Gln Ile Gly Lys Pro Ile Lys Pro Met Leu Ala Gln Gln
   545 550 555 560
   Ala Ala Asn Ile Lys Glu Ala Leu Leu Glu Met Gly Gly Glu Ala Glu
   565 570 575
   Phe Glu Ile Lys Tyr Asp Gly Ala Arg Val Gln Val His Lys Asp Gly
   580 585 590
   Asp Lys Ile Ile Val Tyr Ser Arg Arg Leu Glu Asn Val Thr Arg Ala
   595 600 605
   Ile Pro Glu Ile Val Glu Ala Leu Lys Gln Ser Val Lys Pro Asn Lys
   610 615 620
   Ala Ile Val Glu Gly Glu Leu Val Ala Ile Gly Glu Asp Gly Arg Pro
   625 630 635 640
   Leu Pro Phe Gln Tyr Val Leu Arg Arg Phe Arg Arg Lys His Asn Ile
   645 650 655
   Gln Glu Met Met Lys Lys Ile Pro Leu Glu Leu Asn Leu Phe Asp Val
   660 665 670
   Leu Tyr Val Asp Gly Glu Ser Met Ile Asp Val Lys Phe Ile Asp Arg
   675 680 685
   Arg Lys Lys Leu Glu Glu Ile Ile Glu Pro Asn Gly Lys Ile Lys Val
   690 695 700
   Ala Glu Asn Leu Ile Thr Lys Lys Val Glu Glu Ala Glu Ala Phe Tyr
   705 710 715 720
   Lys Lys Ala Leu Glu Met Gly His Glu Gly Leu Met Ala Lys Arg Leu
   725 730 735
   Asp Ala Thr Tyr Glu Pro Gly Asn Arg Gly Lys Lys Trp Leu Lys Ile
   740 745 750
   Lys Pro Thr Met Glu Asn Leu Asp Leu Val Ile Ile Gly Ala Glu Trp
   755 760 765
   Gly Glu Gly Arg Arg Ala His Leu Leu Gly Ser Phe Ile Leu Gly Ala
   770 775 780
   Tyr Asp Pro Glu Thr Gly Glu Phe Leu Glu Val Gly Lys Val Gly Ser
   785 790 795 800
   Gly Phe Thr Asp Glu Asp Leu Val Glu Phe Thr Lys Met Leu Lys Pro
   805 810 815
   Leu Ile Ile Lys Glu Glu Gly Lys Arg Val Trp Ile Glu Pro Lys Ile
   820 825 830
   Val Ile Glu Val Thr Tyr Gln Glu Ile Gln Lys Ser Pro Lys Tyr Lys
   835 840 845
   Ser Gly Phe Ala Leu Arg Phe Pro Arg Tyr Val Ala Leu Arg Asp Asp
   850 855 860
   Lys Gly Pro
   865
<210> 20
   <211> 49
   <212> DNA
   <213> Artificial Sequence
<220>
   <223> Description of Artificial Sequence: Synthetic oligonucleotide
<220>
   <221> modified_base
   <222> (1)..(1)
   <223> 5Phos-a
<400> 20
   aattctcttt aaataaaccc aaggtctcag atttcatgca gattgtgtc 49
<210> 21
   <211> 49
   <212> DNA
   <213> Artificial Sequence
<220>
   <223> Description of Artificial Sequence: Synthetic oligonucleotide
<220>
   <221> modified_base
   <222> (1)..(1)
   <223> 5Phos-g
<400> 21
   gacacaatct gcatgaaatc tgagaccttg ggtttattta aagagaatt 49
<210> 22
   <211> 5
   <212> PRT
   <213> Unknown
<220>
   <223> Description of Unknown: Unknown natural target p50 sequence
<400> 22
   Ala Asp Gly Pro Tyr
   1 5
<210> 23
   <211> 5
   <212> PRT
   <213> Unknown
<220>
   <223> Description of Unknown: Unknown natural target p50 sequence
<400> 23
   Arg Lys Arg Gln Lys
   1 5

## Description

### BACKGROUND

A variety of methods for the amplification of nucleic acids are known. For example, polymerase chain reaction ("PCR") (see, e.g. U.S. Patent No. 4,683,202) is a popular method for the amplification of nucleic acids. To successfully perform a PCR reaction, the reaction must be performed at multiple different temperatures. This requires hardware or other mechanisms for repeatedly changing the temperature of the PCR reaction. Another method for amplification of nucleic acids is referred to as loop-mediated isothermal amplification ("LAMP") (see, e.g. U.S. Patent No. 6,410,278). LAMP reactions may be performed isothermally, but typically involve the use of four different primers which recognize a total of six distinct sequences on the target nucleic acid.

Some amplification methods utilize DNA ligation. DNA ligation is a common molecular biology method for joining multiple DNA fragments. Ligases can seal single-strand nicks in duplex DNA, join two pieces with complementary "sticky" ends, and in some cases join two pieces with blunt, non-sticky ends. These methods find use in molecular cloning, nucleic acid diagnostics/detection, nucleic acid amplification, and related activities. Ligases and ligation methods are described, for example, in U.S. Patent Application Publication No. 20120214208; U.S. Patent 7,927,853; de Lumley et al., J. Mol. Biol. (2004) 339, 1059-1075; and Rolland et al., FEMS Microbiol. Lett. (2004) 236, 267-273_{.} WO2011/034449 discloses fusion proteins between DNA binding domains and DNA-ligases able to perform blunt-end ligations. Lauer et al., (1991), Journal of Bacteriology, 173(3), 5047-5053, discloses a natural thermostable ligase. Also WO94/02615 discloses such a thermostable ligase.

Ligation of two strands of "sticky-ended" DNA is possible, where the substrates to be joined are already pre-positioned in the case of single-strand nick sealing, and the affinity of sticky ends for each other helps drive sticky-end ligation. However, since DNA ligation depends upon the juxtaposition of the two substrates to be joined, DNA ligation is more difficult in situations where the juxtaposition of the two substrates is more difficult, or the strands are likely to become misaligned or to separate readily. For this reason, blunt-end and high temperature ligations are two types of ligation that are particularly difficult. Blunt-end ligation, however, depends upon random interactions of the substrates. Two adjustments in blunt-end ligation are commonly made to drive the substrate interactions: low temperature to slow down molecular motion so that random interactions last longer and thus give the ligase a better chance to join the fragments; and molecular crowding reagents such as polyethylene glycol to increase the local concentrations of substrates. Likewise, high temperature ligations are challenging because the interactions of DNA substrates are briefer. The least efficient case is thus a high temperature blunt-end ligation in which molecular crowding reagents cannot be used.

A thermostable ligase, T4 DNA ligase, has been described that can perform blunt-end ligations; however, it is inactivated at temperature above approximately 45°C, so that the range of temperature at which T4 DNA ligase is stable is relatively small. A few other examples of ligases that can be induced to join blunt-ended fragments are known, but these ligases appear to do so only in the presence of high concentrations of molecular crowding agents such as 50% polyethylene glycol (PEG). However, the utility of ligases that require such molecular crowding agents is unclear, since 50% PEG which DNA polymerization (which is required for DNA amplification).

Accordingly, in order to facilitate the generation of amplified nucleic acids for the many and growing number of applications which use amplified nucleic acids, new methods and reagents for the amplification of nucleic acids are desired.

### SUMMARY

The present invention provides a method for performing a thermostable blunt-end ligase reaction according to claim 1 and its dependent claims. The "embodiments" disclosed in the following paragraphs are not to be considered embodiments of the present invention, unless they fall within the scope of the claims, but rather are aspects of this disclosure that are useful in understanding the invention.

Fusion proteins having thermostable blunt-end ligase activity are provided. Such thermostable blunt-end ligases are useful for DNA amplification, sequencing, production of recombinant DNA (e.g., as a result of such blunt-end fusions) and recombinant fusion proteins (e.g., proteins encoded by recombinant DNA produced as a result of such blunt-end fusions), and for other purposes. Many molecular biology schemes involve ligases; such schemes would benefit from the ability to perform such schemes at elevated temperatures. Thermostable blunt-end DNA ligases disclosed herein are suitable for use in ligation schemes which would benefit from at least one elevated temperature incubation, such as an incubation at about 60-65°C or higher, including incubation schemes at temperatures as high as about 94°C. A thermostable blunt-end ligase as disclosed herein would be useful for such schemes

In addition, thermostable blunt-end ligases as disclosed herein may enable high temperature blunt-end ligation without the need for molecular crowding agents. Accordingly, thermostable blunt-end ligase as disclosed herein may be useful for many nucleic acid ligation-amplification schemes, including nucleic acid ligation-amplification schemes which operate at a uniform temperature (e.g., at about 60°C or higher), and including nucleic acid ligation-amplification schemes which require temperature cycling such as cycling from, e.g., about 94°C to about 60°C (or higher) for one, two, three, or more cycles. Nucleic acid ligation-amplification schemes which may benefit from the use of thermostable blunt-end DNA ligases disclosed herein may operate at other temperatures as well, e.g., depending on the temperature resistance characteristics of the novel fusion proteins disclosed herein and the requirements of the difference nucleic acid ligation-amplification schemes. Thermostable blunt-end DNA ligases disclosed herein provide the ability to use high temperatures in nucleic acid ligation-amplification schemes and thereby enable higher specificity target amplification, for example, by permitting temperature denaturation of double-stranded DNA templates as well as specific primer binding.

Accordingly, Applicant discloses a thermostable blunt-end nucleic acid ligase comprising a fusion protein comprising a nucleic acid ligase and a nucleic acid binding protein, wherein said thermostable blunt-end nucleic acid ligase is suitable for use in a blunt-ended nucleic acid ligation reaction performed at an elevated temperature. In embodiments, an elevated temperature comprises a temperature of 60 °C or higher. In embodiments, an elevated temperature comprises a temperature of about 65 °C, or about 70 °C, or about 75 °C, or about 80 °C, or about 85 °C, or about 90 °C, or about 95 °C, or higher. Applicant further discloses an article of manufacture, comprising such a thermostable blunt-end nucleic acid ligase, and a container. In embodiments, the article of manufacture further comprises a buffer. Applicant further discloses a method of ligating blunt-end nucleic acids at an elevated temperature, comprising using a thermostable blunt-end nucleic acid ligase as disclosed herein, wherein the use may be at a temperature of about 60 °C or higher. Applicant further discloses a device for analyzing a sample containing nucleic acids, comprising a thermostable blunt-end nucleic acid ligase comprising a fusion protein comprising a nucleic acid ligase and a nucleic acid binding protein, wherein said thermostable blunt-end nucleic acid ligase is suitable for use in a blunt-ended nucleic acid ligation reaction performed at about 60 °C or higher.

In embodiments, Applicant discloses herein a thermostable blunt-end DNA ligase comprising a fusion protein comprising a DNA ligase and a DNA binding protein, wherein said thermostable blunt-end DNA ligase is suitable for use in a blunt-ended DNA ligation reaction performed at an elevated temperature, for example, at a temperature of 60 °C or higher. In embodiments, the thermostable blunt-end DNA ligase comprises a T4 DNA ligase with an N-terminal p50 fusion.

In a particular embodiment, the thermostable blunt-end DNA ligase comprises the amino acid sequence (SEQ ID NO: 1):

A thermostable blunt-end DNA ligase as disclosed herein may comprise a fusion protein which comprises a component selected from a peptide linker, an N-terminal addition, a C-terminal addition, a tag peptide, a D-amino acid, and a peptide mimetic. In embodiments, a thermostable blunt-end DNA ligase as disclosed herein comprises a fusion protein which comprises a component selected from a sugar and a polymer.

In embodiments, a thermostable blunt-end DNA ligase as disclosed herein is suitable for use in a blunt-ended DNA ligation reaction performed at about 75 °C.

In embodiments, a thermostable blunt-end DNA ligase as disclosed herein is capable of making concatamers upon multiple ligation events in a blunt-ended DNA ligation reaction performed at 60 °C or higher.

In embodiments, the thermostable blunt-end DNA ligase as disclosed herein is suitable for use in a nucleic acid amplification scheme which operates at a uniform temperature of 60 °C or higher.

In embodiments, the thermostable blunt-end DNA ligase as disclosed herein is suitable for use in a nucleic acid amplification scheme which comprises temperature cycling. In embodiments, such temperature cycling comprises temperature cycling to temperatures of 60 °C or higher In embodiments, such temperature cycling comprises temperature cycling from about 94 °C to 60 °C. In embodiments, such temperature cycling comprises two or more cycles of temperature cycling, and, in embodiments, comprises three or more cycles of temperature cycling.

Applicant further discloses a method of ligating blunt-end DNA at elevated temperature, comprising using a thermostable blunt-end DNA ligase as disclosed herein, wherein the use mav be at an elevated temperature. In embodiments, an elevated temperature comprises a temperature of 60 °C or higher. In embodiments, an elevated temperature comprises a temperature of about 75 °C; and, in further embodiments, may comprise a temperature higher than about 75 °C.

Applicant discloses herein an article of manufacture, comprising a thermostable blunt-end DNA ligase and a container, wherein the thermostable blunt-end DNA ligase is a thermostable blunt-end DNA ligase as disclosed herein. The article of manufacture further comprises a buffer.

Applicant discloses herein a device for analyzing a sample containing DNA, comprising a thermostable blunt-end DNA ligase, wherein the thermostable blunt-end DNA ligase is a thermostable blunt-end DNA ligase as disclosed herein.

### BRIEF DESCRIPTION OF THE FIGURE

Fig. 1 shows an example of the results of a blunt-ended ligation reaction performed at 75 °C using a thermostable, blunt-end DNA ligase as provided herein. The DNA substrate was a 49-bp duplex DNA made by annealing oligonucleotides EE0139 (SEQ ID NO:20) and EE0140 (SEQ ID NO: 21).

### DETAILED DESCRIPTION

Thermostable, blunt-end ligases are provided herein. As disclosed herein, a thermostable, blunt-end ligase may be prepared by the fusion of a DNA-binding protein to a thermostable ligase. Such ligases produced by these fusions have features and capabilities not provided by their parent compounds alone; the different portions of these fusion proteins provide activities which, when combined, provide new capabilities and unexpectedly improved activity. The DNA-binding protein portion of such fusion proteins is effective to increase the affinity of the ligase to DNA substrates, resulting in enhanced ligation in the challenging conditions of high temperature, blunt-end ligation. The DNA ligase portion surprisingly retains its ability to ligate DNA when combined with a foreign protein (the DNA-binding protein portion). Together, the combined portions unite in novel fusion proteins that are able to ligate blunt-ended DNA substrates even at high temperatures, providing increased ligation activity at high temperatures unavailable by the use of the original, unmodified ligases.

Methods, reagents, devices, systems, and articles of manufacture useful for the practice of the methods, and for the use of reagents, devices, systems, and articles of manufacture disclosed herein, are described, for example, in WO 2014/145296.

### DEFINITIONS

Before the present novel ligases and ligation methods are disclosed and described, it is to be understood that the terminology used herein is for the purpose of describing particular embodiments only and is not intended to be limiting. It is also to be understood that the present disclosure provides explanatory and exemplary descriptions and examples, so that, unless otherwise indicated, the molecules, compositions, assays, methods, and kits disclosed herein are not limited to the specific embodiments described herein.

It must be noted that, as used in the specification and the appended claims, the singular forms "a," "an" and "the" include plural referents unless the context clearly dictates otherwise. Thus, for example, reference to "a salt" refers to a single salt or mixtures of different salts, and the like.

As used in the description herein and throughout the claims that follow, the meaning of "in" includes "in" and "on" unless the context clearly dictates otherwise.

As used in the description herein and throughout the claims that follow, the meaning of "or" includes both the conjunctive and disjunctive unless the context expressly dictates otherwise. Thus, the term "or" includes "and/or" unless the context expressly dictates otherwise.

In this specification and in the claims that follow, reference will be made to a number of terms, which shall be defined to have the following meanings:

The term "moiety" as used herein refers to any particular composition of matter, e.g., a molecular fragment, an intact molecule, or a mixture of materials.

The term "nucleic acid" refers to nucleotides and nucleosides which make up, for example, deoxyribonucleic acid (DNA) macromolecules and ribonucleic acid (RNA) macromolecules. Nucleic acids may be identified by the base attached to the sugar (e.g., deoxyribose or ribose); as used herein, the following abbreviations for these bases are used to represent nucleic acids in sequence listings identifying and describing their structures (either upper-case or lower-case may be used).

**TABLE 1A**

| Base (in Nucleic Acid) | Letter Code |
|---|---|
| Adenine | A |
| Thymine | T |
| Guanine | G |
| Cytosine | c |
| Uracil | U |

As used herein, a "polynucleotide" refers to a polymeric chain containing two or more nucleotides. "Polynucleotides" includes primers, oligonucleotides, nucleic acid strands, etc. A polynucleotide may contain standard or non-standard nucleotides. Typically, a polynucleotide contains a 5' phosphate at one terminus ("5' terminus") and a 3' hydroxyl group at the other terminus ("3' terminus) of the chain. The most 5' nucleotide of a polynucleotide may be referred to herein as the "5' terminal nucleotide" of the polynucleotide. The most 3' nucleotide of a polynucleotide may be referred to herein as the "3' terminal nucleotide" of the polynucleotide.

The term "downstream" as used herein in the context of a polynucleotide containing a 5' terminal nucleotide and a 3' terminal nucleotide refers to a position in the polynucleotide which is closer to the 3' terminal nucleotide than a reference position in the polynucleotide. For example, in a primer having the sequence: 5' ATAAGC 3', the "G" is downstream from the "T" and all of the "A"s.

The term "upstream" as used herein in the context of a polynucleotide containing a 5' terminal nucleotide and a 3' terminal nucleotide, refers to a position in the polynucleotide which is closer to the 5' terminal nucleotide than a reference position in the polynucleotide. For example, in a primer having the sequence: 5' ATAAGC 3', the "T" is upstream from the "G", the "C", and the two "A"s closest to the "G".

As used herein, "nucleic acid" includes both DNA and RNA, including DNA and RNA containing non-standard nucleotides. A "nucleic acid" contains at least one polynucleotide (a "nucleic acid strand"). A "nucleic acid" may be single-stranded or double-stranded.

As used herein, a nucleic acid molecule which is described as containing the "sequence" of a template or other nucleic acid may also be considered to contain the template or other nucleic acid itself (e.g. a molecule which is described as containing the sequence of a template may also be described as containing the template), unless the context clearly dictates otherwise.

As used herein, a "target" nucleic acid or molecule refers to a nucleic acid of interest. A target nucleic acid / molecule may be of any type, including single-stranded or double stranded DNA or RNA (e.g. mRNA).

As used herein, "complementary" sequences refer to two nucleotide sequences which, when aligned anti-parallel to each other, contain multiple individual nucleotide bases which pair with each other. It is not necessary for every nucleotide base in two sequences to pair with each other for sequences to be considered "complementary". Sequences may be considered complementary, for example, if at least 30%, 40%, 50%, 55%, 60%, 65%, 70%, 75%, 80%, 85%, 90%, 95%, 98%, 99%, or 100% of the nucleotide bases in two sequences pair with each other. In addition, sequences may still be considered "complementary" when the total lengths of the two sequences are significantly different from each other. For example, a primer of 15 nucleotides may be considered "complementary" to a longer polynucleotide containing hundreds of nucleotides if multiple individual nucleotide bases of the primer pair with nucleotide bases in the longer polynucleotide when the primer is aligned anti-parallel to a particular region of the longer polynucleotide.

As used herein, the term "isolated" as applied to proteins, nucleic acids, or other biomolecules refers to a molecule that has been purified or separated from a component of its naturally-occurring environment (e.g. a protein purified from a cell in which it was naturally produced). An "isolated" molecule may be in contact with other molecules (for example, as part of a reaction mixture). As used herein, "isolated" molecules also include recombinantly-produced proteins or nucleic acids which have an amino acid or nucleotide sequence which occurs naturally. "Isolated" nucleic acids include polypeptide-encoding nucleic acid molecules contained in cells that ordinarily express the polypeptide where, for example, the nucleic acid molecule is at a chromosomal location different from that of natural cells. In some embodiments, "isolated" polypeptides are purified to at least 50 %, 60%, 70%, 80%, 90%, 95%, 98%, or 100% homogeneity as evidenced by SDS-PAGE of the polypeptides followed by Coomassie blue, silver, or other protein staining method.

The terms "polypeptide" and "protein" may be used interchangeably to refer to molecules comprised of amino acids linked by peptide bonds. Individual amino acids may be termed "residues" of a polypeptide or protein. The amino acid sequences of polypeptides disclosed herein may be identified by SEQ ID NO: presented as a string of letters, where the letters have the following meanings:

**TABLE 1B**

| Amino Acid | 3-Letter Code | 1-Letter Code |
|---|---|---|
| Alanine | Ala | A |
| Arginine | Arg | R |
| Asparagine | Asn | N |
| Aspartic acid | Asp | D |
| Cysteine | Cys | c |
| Glutamic acid | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | s |
| Threonine | Thr | T |
| Tryptophan | Trp | w |
| Tyrosine | Tyr | Y |
| Valine | Val | V |

Naturally occurring amino acid residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

"Identical" or "identity," as used herein in the context of two or more polypeptide or polynucleotide sequences, can mean that the sequences have a specified percentage of residues that are the same over a specified region. The percentage can be calculated by optimally aligning the two sequences, comparing the two sequences over the specified region, determining the number of positions at which the identical residue occurs in both sequences to yield the number of matched positions, dividing the number of matched positions by the total number of positions in the specified region, and multiplying the result by 100 to yield the percentage of sequence identity. In cases where the two sequences are of different lengths or the alignment produces one or more staggered ends and the specified region of comparison includes only a single sequence, the residues of the single sequence are included in the denominator but not the numerator of the calculation.

"Homology" or "homologous" as used herein in the context of two or more polypeptide or polynucleotide sequences, can mean that the sequences have a specified percentage of residues that are either i) the same, or ii) conservative substitutions of the same residue, over a specified region. Conservative substitutions include substitutions of one amino acid by an amino acid of the same group, and include substitutions of one amino acid by an amino acid identified in Table 1C as an exemplary or as a preferred substitution. In determining homology of two sequences, identical residues and homologous residues are given equal weight. The percentage can be calculated by optimally aligning the two sequences, comparing the two sequences over the specified region, determining the number of positions at which either identical or homologous residues occur in both sequences to yield the number of matched positions, dividing the number of matched positions by the total number of positions in the specified region, and multiplying the result by 100 to yield the percentage of sequence homology. In cases where the two sequences are of different lengths or the alignment produces one or more staggered ends and the specified region of comparison includes only a single sequence, the residues of the single sequence are included in the denominator but not the numerator of the calculation.

Variant or modified polypeptides may be prepared by substituting one or more amino acid residues by a different one or more amino acid residues in the amino acid sequence of the polypeptide. Conservative substitutions will entail exchanging one member of a group for another member of the same group; such changes tend not to significantly affect the function of a polypeptide. Non-conservative substitutions will entail exchanging a member of one of these classes for another group. Substantial modifications of polypeptides may be accomplished without significantly affecting the functional characteristics of a polypeptide by selecting substitutions that maintain one or more of (a) the structure of the polypeptide backbone in the area of the substitution, for example, as a sheet or helical conformation, (b) the charge or hydrophobicity of the molecule at the target site, or (c) the bulk of the side chain.

In particular embodiments, conservative substitutions of interest are shown in Table 1C under the headings of "exemplary substitutions" and "preferred substitutions." Such conservative substitutions may not result in significant changes in biological activity.

**TABLE 1C**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Further substitutions may be performed, and suitable substitutions may be identified by scanning amino acid analysis, in which a single amino acid, or a very few amino acids, of an initial sequence are replaced. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main chain conformation of the variant [Cunningham and Wells, Science, 244: 1081-1085 (1989)].

In addition, one or more amino acids in a sequence may be substituted by a non-conventional amino acid. Conservative replacements comprising non-conventional amino acids substitute the original amino acid with a non-conventional amino acid that resembles the original in one or more of its, characteristic properties (e.g., charge, hydrophobicity, stearic bulk; for example, one may replace Val with Nval). The term "non-conventional amino acid" refers to amino acids other than conventional amino acids, and includes, for example, isomers and modifications of the conventional amino acids (e.g., D-amino acids), non-protein amino acids, post-translationally modified amino acids, enzymatically modified amino acids, constructs or structures designed to mimic amino acids (e.g., .alpha.,.alpha.-disubstituted amino acids, N-alkyl amino acids, lactic acid, .beta.-alanine, naphthylalanine, 3-pyridylalanine, 4-hydroxyproline, O-phosphoserine, N-acetylserine, N-formylmethionine, 3-methylhistidine, 5-hydroxylysine, and norleucine), and peptides having the naturally occurring amide --CONH-- linkage replaced at one or more sites within the peptide backbone with a non-conventional linkage such as N-substituted amide, ester, thioamide, retropeptide (--NHCO--), retrothioamide (--NHCS--), sulfonamido (--SO.sub.2 NH--), and/or peptoid (N-substituted glycine) linkages.

Homologous proteins and other protein variants may be provided substitution, insertion, deletion, or addition. Such substitution, insertion, deletion, or addition may include substitution, insertion, deletion, or addition of a single residue; may include substitution, insertion, deletion, or addition of two residues; and may include substitution, insertion, deletion, or addition of three, or of more, residues. For example, substitution may include replacing one, two, three, four, five, or more amino acid residues by a different residue. Substitution may be, or may include, conservative substitution. For example, insertion may include inserting one, two, three, four, five, or more amino acid residues into an amino acid sequence. For example, deletion may include deleting one, two, three, four, five, or more amino acid residues from an amino acid sequence. For example, addition may include adding one, two, three, four, five, or more amino acid residues at the N-terminal end, at the C-terminal end, or both, of an amino acid sequence.

A composition may include a buffer. Buffers include, without limitation, phosphate, citrate, ammonium, acetate, carbonate, tris(hydroxymethyl)aminomethane (TRIS), 3-(N-morpholino) propanesulfonic acid (MOPS), 3-morpholino-2-hydroxypropanesulfonic acid (MOPSO), 2-(*N-*morpholino)ethanesulfonic acid (MES), N-(2-Acetamido)-iminodiacetic acid (ADA), piperazine-N,N'-bis(2-ethanesulfonic acid) (PIPES), N-(2-Acetamido)-2-aminoethanesulfonic acid (ACES), cholamine chloride, N,N-Bis(2-hydroxyethyl)-2-aminoethanesulfonic acid (BES), 2-[[1,3-dihydroxy-2-(hydroxymethyl)propan-2-yl]amino]ethanesulfonic acid (TES), 4-(2-hydroxyethyl)-1-piperazine ethanesulfonic acid (HEPES), acetamidoglycine, tricine (N-(2-Hydroxy-1,1-bis(hydroxymethyl)ethyl)glycine), glycinamide, and bicine (2-(Bis(2-hydroxyethyl)amino)acetic acid) buffers. Buffers include other organic acid buffers in addition to the phosphate, citrate, ammonium, acetate, and carbonate buffers explicitly mentioned herein.

An article of manufacture may comprise a container; and a composition contained within the container, wherein the composition comprises a thermostable ligase. An article of manufacture may comprise a container; and a composition contained within the container, wherein the composition comprises a blunt-end ligase. An article of manufacture may comprise a container; and a composition contained within the container, wherein the composition comprises a thermostable blunt-end ligase. The containers may be formed from a variety of materials such as glass or plastic, and may have a sterile access port (for example the container may be an intravenous solution bag or a vial having a stopper pierceable by a hypodermic injection needle). The article of manufacture may further comprise a label or package insert on or associated with the container indicating that the composition may be used to ligate blunt-ended DNA.

Embodiments of methods provided herein may be described with reference to Figure 1.

The novel thermostable blunt-end DNA ligases disclosed herein are comprised of the fusion of a DNA-binding protein with a thermostable ligase. Such fusion proteins as disclosed herein may further comprise other components, such as, for example, peptide linkers, N-terminal or C-terminal additions, tag peptides, and other amino acid sequences, including D-amino acids and peptide mimetics. In addition, fusion protein as disclosed herein may further comprise other components, such as, for example, sugars (e.g., the fusion proteins may be glycosylated), polymers such as polyethylene glycol (e.g., the fusion proteins may be PEGylated), organic moieties other than amino acids, and other additions linked to the fusion proteins. The DNA-binding protein portion increases the affinity of the ligase to DNA substrates, resulting in enhanced ligation in the challenging conditions of high temperature, blunt-end ligation. Many combinations of binding proteins and ligases are possible. For example, the binding protein may be N-terminal or C-terminal relative to the ligase.

It will be understood that multiple combinations of DNA ligases and DBA-binding proteins may be joined to produce the fusion proteins disclosed herein. For example, the following 16 combinations have been constructed (Table 2):

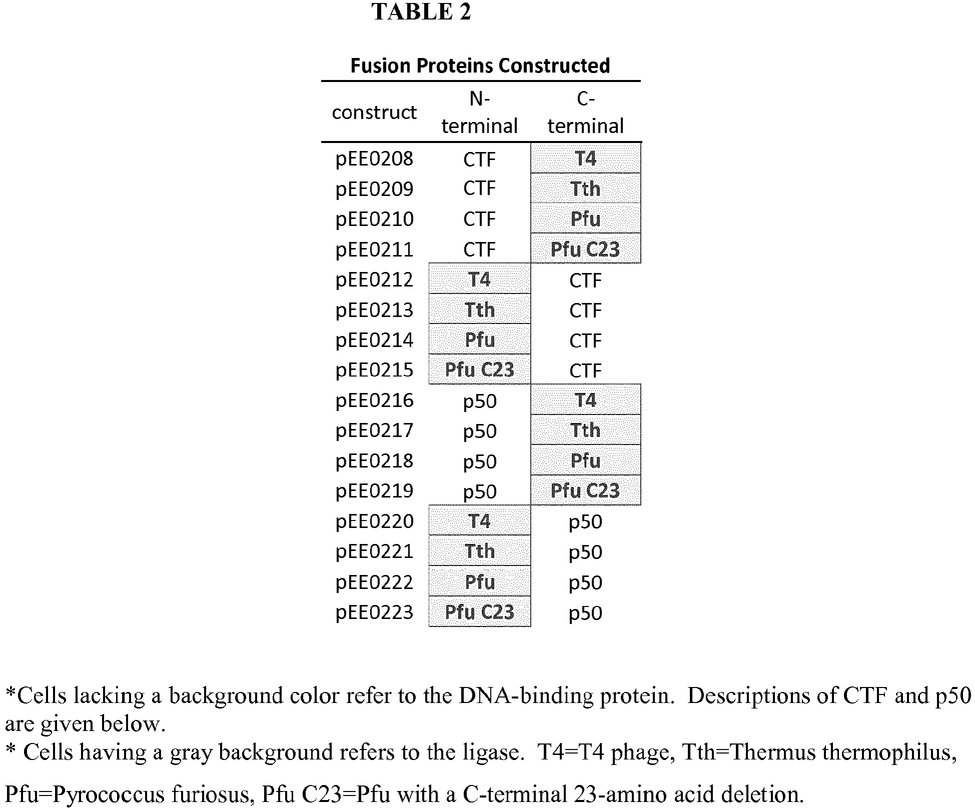

Additional variations are possible, including: (1) different DNA-binding proteins, including thermostable proteins; (2) other DNA ligases, including T4 DNA ligase variants engineered to be thermostable; (3) multiple DNA-binding proteins per ligase, or multiple ligases per DNA-binding protein; (4) transient, non-covalent linkages between the ligase and DNA-binding protein, rather than a protein fusion, to better enable multiple ligation events per ligase molecule; (5) dimers or higher-order multimers of fusion proteins to increase the local concentration of ligase once DNA substrates are bound; (6) different degrees of affinity between the DNA substrate and the DNA-binding protein - for example, using the natural target sequence of p50, GGGAATTCCC (SEQ ID NO: 6), in the DNA target, to enable low picomolar affinity; (7) other nucleic acid modifying enzymes instead of DNA ligase to perform other molecular reactions.

An exemplary method for producing the fusion proteins disclosed herein may be as follows. Fusion proteins may be made by inducing E. coli to express DNA constructs made by standard recombinant DNA methods, followed by standard chromatographic protein purification methods. An affinity tag such as polyhistidine may be employed to assist in the protein purification. In the case of thermostable proteins, purification may be assisted by employing heat to denature most E. coli host proteins. The purified fusion protein may be used in the same way that a standard, non-heat-stable ligase would be used in the application/reaction of choice, for example in a scheme that depends upon ligation of DNA substrates to make a template for amplification.

The methods disclosed herein can be readily incorporated into and used in device for processing a sample, or a system for processing a sample, which may be an automated assay device, or may be an automated assay system. Such assay devices and assay systems may comprise devices and systems disclosed, for example, in U.S. Patent 8,088,593; U.S. Patent 8,380,541; U.S. Pat. Pub. No. US 2013/0166343; WO 2013/052318A1, filed September 25, 2012; U.S. Patent Pub. No. US 2013/0078624, filed September 26, 2011; and WO 2014/015199, filed September 26, 2011.

Assay devices and assay systems, including devices for processing a sample and systems for processing a sample, which may be automated assay devices and automated assay systems, may be located and may be used at a point of care location, and may be located and may be used at a plurality of point of care locations. Assay devices and assay systems, including devices for processing a sample and systems for processing a sample, which may be automated assay devices and automated assay systems, may be located and may be used at a point of care location, and may be located and may be used at a plurality of point of service locations.

A sample processing device for performing assays and measurements as disclosed herein may be located, and may perform such assays and measurements, at a point of care location, or a point of service location, or other location. A sample processing system for performing assays and measurements as disclosed herein may be located, and may perform such assays and measurements, at a point of care location, or a point of service location, or other location.

A point of care system may be used at a point of service location, such as a subject's location (e.g., home or business or sports event or security screening or combat location), the location of a healthcare provider (e.g., doctor), a pharmacy or retailer, a clinic, a hospital, an emergency room, a nursing home, a hospice care location, or a laboratory. A retailer may be a pharmacy (e.g., retail pharmacy, clinical pharmacy, hospital pharmacy), drugstore, chain store, supermarket, or grocer. Examples of retailers may include but are not limited to Walgreen's, CVS Pharmacy, Duane Reade, Walmart, Target, Rite Aid, Kroger, Costco, Kaiser Permanente, or Sears. In some situations, a point of service system (including but not limited to point of care system) is deployed at any location that is designated for use by a certifying or licensing entity (e.g., a government certifying entity). In other situations, a point of service system may be used in or embedded in a transportation vehicle, such as a car, boat, truck, bus, airplane, motorcycle, van, traveling medical vehicle, mobile unit, ambulance, fire engine/truck, critical care vehicle, or other vehicle configured to transport a subject from one point to another. A sample collection site may be at a sample acquisition site and/or health assessment and/or treatment locations (which may include any of the sample collection sites described elsewhere herein including but not limited to emergency rooms, doctors' offices, urgent care, tents for screening (which may be in remote locations), a health care professional walking into someone's house to provide home care).

The system (device) or a combination of systems (devices) may be located/positioned at strategic point of service locations. Locations may be selected and optimized based on a variety of objectives, such as but not limited to disease prevalence, rates of disease development, projected disease rates, estimated risk of outbreaks, population demographics, government policies and regulations, customer, physician and patient preferences, access to other technologies at said locations, safety and risk estimates, safety threats, etc. Devices can be relocated on a periodic basis to improve overall utility on a frequent basis, such as daily, weekly, monthly, annually, etc. Systems can be updated to improve performance and/or add functionality. Systems can be updated on a module by module basis. System updates can occur via hardware and/or via software. Systems can be updated with minimal downtime via features enabling blade and/or module extraction and insertion.

Additionally, a point of service location where a sample may be collected from a subject or provided by a subject may be a location remote to an analyzing laboratory. The sample collection site may have a separate facility from the laboratory. The sample may or may not be collected fresh from the subject at the point of service location. Alternatively, the sample may be collected from the subject elsewhere and brought to the point of service location. In some embodiments, no sample preparation step is provided on the sample before being provided to the device. For example, no slide needs to be prepped before a sample is provided to the device. Alternatively, one or more sample preparation step may be performed on the sample before being provided to the device.

A sample collection site at a point of service location may be a blood collection center, or any other bodily fluid collection center. The sample collection site may be a biological sample collection center. In some embodiments, a sample collection site may be a retailer. Other examples of sample collection sites may include hospitals, clinics, health care professionals' offices, schools, day-care centers, health centers, assisted living residences, government offices, traveling medical care units, or the home. For example, a sample collection site may be a subject's home. A sample collection site may be any location where a sample from the subject is received by the device. A collection site may be a moving location, such as on or with a patient or in a mobile unit or vehicle or with a travelling doctor. Any location may be designated as a sample collection site. The designation may be made by any party, including but not limited to the laboratory, entity associated with the laboratory, governmental agency, or regulatory body. Any description herein relating to sample collection site or point of service location may relate to or be applied to retailers, hospitals, clinics, or any other examples provided herein and vice versa.

A device may be part of a system, a component of which may be a sample processing device. A device may be a sample processing device. A sample processing device may be configured to facilitate collection of a sample, prepare a sample for a clinical test, or effect a chemical reaction with one or more reagents or other chemical or physical processing, as disclosed herein. A sample processing device may be configured to obtain data from a sample. A sample processing device may be configured to transmit data obtained from a sample. A sample processing device may be configured to analyze data from a sample. A sample processing device may be configured to communicate with another device, or a laboratory, or an individual affiliated with a laboratory, to analyze data obtained from a sample.

Point of service systems described herein are configured to process samples at a location where the point of service system is accessible by a user. In an example, a point of service system is located at a subject's home and a sample is collected from a subject and processed in the subject's home. In another example, a point of service system is located at a drug store and a sample is collected from a subject and processed in the drug store. In another example, a point of service system is located at the location of a healthcare provider (e.g., doctor's office) and a sample is collected from a subject and processed at the location of the healthcare provider. In another example, a point of service system is located onboard a transportation system (e.g., vehicle) and a sample is collected from a subject and processed on the transportation system.

In some examples, post-sample processing analysis, including diagnosis and/or treatment, is performed by the point of service system at the location of the point of service system. In other examples, post-sample processing analysis is performed remotely from a location in which a sample is collected and processed. In an example, post-sample processing analysis is performed at the location of a healthcare provider. In another example, post-sample processing analysis is performed at the location of a processing system. In another example, post-sample processing analysis is performed on a server (e.g., on the cloud).

The post-sampling analysis may occur at a laboratory or by an entity affiliated with a laboratory. A laboratory can be an entity or facility capable of performing a clinical test or analyzing collected data. A laboratory can provide controlled conditions in which scientific research, experiments, and measurement can be performed. The laboratory can be a medical laboratory or clinical laboratory where tests can be done on clinical specimens, or analysis can occur on data collected from clinical specimens, in order to get information about the health of a patient as pertaining to the diagnosis, prognosis, treatment, and/or prevention of disease. A clinical specimen may be a sample collected from a subject. Preferably, a clinical specimen may be collected from the subject at a sample collection site that is at a separate facility from the laboratory, as described in further detail elsewhere herein. The clinical specimen may be collected from the subject using a device, which is placed at a designated sample collection site or in or on the subject.

A sample processing device may be configured to be placed in or on a subject. A sample processing device may be configured to accept a sample from a subject, either directly or indirectly. A sample may be, for example, a blood sample (e.g., a sample obtained from a fingerstick, or from venipuncture, or an arterial blood sample), a urine sample, a biopsy sample, a tissue slice, stool sample, or other biological sample; a water sample, a soil sample, a food sample, an air sample; or other sample. A blood sample may comprise, e.g., whole blood, plasma, or serum. A sample processing device may receive a sample from the subject through a housing of the device. The sample collection may occur at a sample collection site, or elsewhere. The sample may be provided to the device at a sample collection site.

In some examples, a sample processing device may be configured to accept or hold a cartridge. In some examples, a sample processing device may comprise a cartridge. The cartridge may be removable from the sample processing device. In some examples, a sample may be provided to the cartridge of the sample processing device. Alternatively, a sample may be provided to another portion of a sample processing device. The cartridge and/or device may comprise a sample collection unit that may be configured to accept a sample.

A cartridge may include a sample, and may include reagents for use in processing or testing a sample, disposables for use in processing or testing a sample, or other materials. Following placement of a cartridge on, or insertion of a cartridge into, a sample processing device, one or more components of the cartridge may be brought into fluid communication with other components of the sample processing device. For example, if a sample is collected at a cartridge, the sample may be transferred to other portions of the sample processing device. Similarly, if one or more reagents are provided on a cartridge, the reagents may be transferred to other portions of the sample processing device, or other components of the sample processing device may be brought to the reagents. In some examples, the reagents or components of a cartridge may remain on-board the cartridge. In some examples, no fluidics are included that require tubing or that require maintenance (e.g., manual or automated maintenance).

A sample or reagent may be transferred to a device, such as a sample processing device. A sample or reagent may be transferred within a device. Such transfer of sample or reagent may be accomplished without providing a continuous fluid pathway from cartridge to device. Such transfer of sample or reagent may be accomplished without providing a continuous fluid pathway within a device. In examples, such transfer of sample or reagent may be accomplished by a sample handling system (e.g., a pipette); for example, a sample, reagent, or aliquot thereof may be aspirated into an open-tipped transfer component, such as a pipette tip, which may be operably connected to a movable unit which transfers the tip, with the sample, reagent, or aliquot thereof contained within the tip, to a location on or within the sample processing device. In some examples, a tip from the sample handling system may be inserted at least partially into the sample vessel and/or cavity. The tip may be insertable and removable from the sample vessel and/or cavity. The sample, reagent, or aliquot thereof can be deposited at a location on or within the sample processing device. Sample and reagent, or multiple reagents, may be mixed using a sample handling system in a similar manner. One or more components of the cartridge may be transferred in an automated fashion to other portions of the sample processing device, and vice versa.

A device, such as a sample processing device, may have a fluid handling system (which may be part of a sample handling system). A fluid handling system may perform, or may aid in performing, transport, dilution, extraction, aliquotting, mixing, and other actions with a fluid, such as a sample. In some examples, a fluid handling system may be contained within a device housing. A fluid handling system may permit the collection, delivery, processing and/or transport of a fluid, dissolution of dry reagents, mixing of liquid and/or dry reagents with a liquid, as well as collection, delivery, processing and/or transport of non-fluidic components, samples, or materials. The fluid may be a sample, a reagent, diluent, wash, dye, or any other fluid that may be used by the device, and may include, but not limited to, homogenous fluids, different liquids, emulsions, suspensions, and other fluids. A fluid handling system, including without limitation a pipette, may also be used to transport vessels (with or without fluid contained therein) around the device. The fluid handling system may dispense or aspirate a fluid. The sample may include one or more particulate or solid matter floating within a fluid.

In examples, a fluid handling system may comprise a pipette, pipette tip, syringe, capillary, or other component. The fluid handling system may have portion with an interior surface and an exterior surface and an open end. The fluid handling system may comprise a pipette, which may include a pipette body and a pipette nozzle, and may comprise a pipette tip. A pipette tip may or may not be removable from a pipette nozzle. In embodiments, a fluid handling system may use a pipette mated with a pipette tip; a pipette tip may be disposable. A tip may form a fluid-tight seal when mated with a pipette. A pipette tip may be used once, twice, or more times. In embodiments, a fluid handling system may use a pipette or similar device, with or without a pipette tip, to aspirate, dispense, mix, transport, or otherwise handle the fluid. The fluid may be dispensed from the fluid handling system when desired. The fluid may be contained within a pipette tip prior to being dispensed, e.g., from an orifice in the pipette tip. In embodiments, or instances during use, all of the fluid may be dispensed; in other embodiments, or instances during use, a portion of the fluid within a tip may be dispensed. A pipette may selectively aspirate a fluid. The pipette may aspirate a selected amount of fluid. The pipette may be capable of actuating stirring mechanisms to mix the fluid within the tip or within a vessel. The pipette may incorporate tips or vessels creating continuous flow loops for mixing, including of materials or reagents that are in non-liquid form. A pipette tip may also facilitate mixture by metered delivery of multiple fluids simultaneously or in sequence, such as in 2-part substrate reactions.

The fluid handling system may include one or more fluidically isolated or hydraulically independent units. For example, the fluid handling system may include one, two, or more pipette tips. The pipette tips may be configured to accept and confine a fluid. The tips may be fluidically isolated from or hydraulically independent of one another. The fluid contained within each tip may be fluidically isolated or hydraulically independent from one fluids in other tips and from other fluids within the device. The fluidically isolated or hydraulically independent units may be movable relative to other portions of the device and/or one another. The fluidically isolated or hydraulically independent units may be individually movable. A fluid handling system may comprise one or more base or support. A base or support may support one or more pipette or pipette units. A base or support may connect one or more pipettes of the fluid handling system to one another.

A sample processing device may be configured to perform processing steps or actions on a sample obtained from a subject. Sample processing may include sample preparation, including, e.g., sample dilution, division of a sample into aliquots, extraction, contact with a reagent, filtration, separation, centrifugation, or other preparatory or processing action or step. A sample processing device may be configured to perform one or more sample preparation action or step on the sample. Optionally, a sample may be prepared for a chemical reaction and/or physical processing step. A sample preparation action or step may include one or more of the following: centrifugation, separation, filtration, dilution, enriching, purification, precipitation, incubation, pipetting, transport, chromatography, cell lysis, cytometry, pulverization, grinding, activation, ultrasonication, micro column processing, processing with magnetic beads, processing with nanoparticles, or other sample preparation action or steps. For example, sample preparation may include one or more step to separate blood into serum and/or particulate fractions, or to separate any other sample into various components. Sample preparation may include one or more step to dilute and/or concentrate a sample, such as a blood sample, or other biological samples. Sample preparation may include adding an anticoagulant or other ingredients to a sample. Sample preparation may also include purification of a sample. In examples, all sample processing, preparation, or assay actions or steps are performed by a single device. In examples, all sample processing, preparation, or assay actions or steps are performed within a housing of a single device. In examples, most sample processing, preparation, or assay actions or steps are performed by a single device, and may be performed within a housing of a single device. In examples, many sample processing, preparation, or assay actions or steps are performed by a single device, and may be performed within a housing of a single device. In embodiments, sample processing, preparation, or assay actions or steps may be performed by more than one device.

A sample processing device may be configured to run one or more assay on a sample, and to obtain data from the sample. An assay may include one or more physical or chemical treatments, and may include running one or more chemical or physical reactions. A sample processing device may be configured to perform one, two or more assays on a small sample of bodily fluid. One or more chemical reaction may take place on a sample having a volume, as described elsewhere herein. For example, one or more chemical reaction may take place in a pill having less than femtoliter volumes. In an instance, the sample collection unit is configured to receive a volume of the bodily fluid sample equivalent to a single drop or less of blood or interstitial fluid. In examples, the volume of a sample may be a small volume, where a small volume may be a volume that is less than about 1000 µL, or less than about 500 µL, or less than about 250 µL, or less than about 150 µL, or less than about 100 µL, or less than about 75 µL, or less than about 50 µL, or less than about 40 µL, or less than about 20 µL, or less than about 10 µL, or other small volume. In examples, all sample assay actions or steps are performed on a single sample. In examples, all sample assay actions or steps are performed by a single device. In examples, all sample assay actions or steps are performed within a housing of a single device. In examples, most sample assay actions or steps are performed by a single device, and may be performed within a housing of a single device. In examples, many sample assay actions or steps are performed by a single device, and may be performed within a housing of a single device. In examples, sample processing, preparation, or assay actions or steps may be performed by more than one device.

A sample processing device may be configured to perform a plurality of assays on a sample. In examples, a sample processing device may be configured to perform a plurality of assays on a single sample. In examples, a sample processing device may be configured to perform a plurality of assays on a single sample, where the sample is a small sample. For example, a small sample may have a sample volume that is a small volume of less than about 1000 µL, or less than about 500 µL, or less than about 250 µL, or less than about 150 µL, or less than about 100 µL, or less than about 75 µL, or less than about 50 µL, or less than about 40 µL, or less than about 20 µL, or less than about 10 µL, or other small volume. A sample processing device may be capable of performing multiplexed assays on a single sample. A plurality of assays may be run simultaneously; may be run sequentially; or some assays may be run simultaneously while others are run sequentially. One or more control assays and/or calibrators (e.g., including a configuration with a control of a calibrator for the assay/tests) can also be incorporated into the device; control assays and assay on calibrators may be performed simultaneously with assays performed on a sample, or may be performed before or after assays performed on a sample, or any combination thereof. In examples, all sample assay actions or steps are performed by a single device. In examples, all of a plurality of assay actions or steps are performed within a housing of a single device. In examples, most sample assay actions or steps, of a plurality of assays, are performed by a single device, and may be performed within a housing of a single device. In examples, many sample assay actions or steps, of a plurality of assays, are performed by a single device, and may be performed within a housing of a single device. In examples, sample processing, preparation, or assay actions or steps may be performed by more than one device.

In examples, all of a plurality of assays may be performed in a short time period. In examples, such a short time period comprises less than about three hours, or less than about two hours, or less than about one hour, or less than about 40 minutes, or less than about 30 minutes, or less than about 25 minutes, or less than about 20 minutes, or less than about 15 minutes, or less than about 10 minutes, or less than about 5 minutes, or less than about 4 minutes, or less than about 3 minutes, or less than about 2 minutes, or less than about 1 minute, or other short time period.

A device may be capable of performing all on-board steps (e.g., steps or actions performed by a single device) in a short amount of time. A device may be capable of performing all on-board steps on a single sample in a short amount of time. For example, from sample collection from a subject to transmitting data and/or to analysis may take about 3 hours or less, 2 hours or less, 1 hour or less, 50 minutes or less, 45 minutes or less, 40 minutes or less, 30 minutes or less, 20 minutes or less, 15 minutes or less, 10 minutes or less, 5 minutes or less, 4 minutes or less, 3 minutes or less, 2 minutes or less, or 1 minute or less. The amount of time from accepting a sample within the device to transmitting data and/or to analysis from the device regarding such a sample may depend on the type or number of steps, tests, or assays performed on the sample. The amount of time from accepting a sample within the device to transmitting data and/or to analysis from the device regarding such a sample may take about 3 hours or less, 2 hours or less, 1 hour or less, 50 minutes or less, 45 minutes or less, 40 minutes or less, 30 minutes or less, 20 minutes or less, 15 minutes or less, 10 minutes or less, 5 minutes or less, 4 minutes or less, 3 minutes or less, 2 minutes or less, or 1 minute or less.

A device or system as disclosed herein, such as, e.g., a point of service device or a point of service system, may process a sample according to a test order or schedule. In some situations, a feedback loop (coupled with sensors) enables the point of service device or system to monitor the progress of sample processing and maintain or alter the test order or schedule. In an example, if the device or system detects that processing is taking longer than the predetermined amount of time set forth in the schedule, the device or system speeds up processing or adjusts any parallel processes, such as sample processing in another module of the device or system. The feedback loop permits real-time or pseudo-real time (e.g., cached) monitoring. In some situations, the feedback loop may provide permit reflex testing, which may cause subsequent tests, assays, preparation steps, and/or other processes to be initiated after starting or completing another test and/or assay or sensing one or more parameter. Such subsequent tests, assays, preparation steps, and/or other processes may be initiated automatically without any human intervention.

In some examples, the point of service system may stick to a pre-determined test order or schedule based on initial parameters and/or desired effects. In other embodiments, the schedule and/or test order may be modified on the fly. The schedule and/or test order may be modified based on one or more detected conditions, one or more additional processes to run, one or more processes to no longer run, one or more processes to modify, one or more resource/component utilization modifications, one or more detected error or alert condition, one or more unavailability of a resource and/or component, one or more subsequent input or sample provided by a user, external data, or any other reason.

In some examples, one or more additional samples may be provided to a device after one or more initial samples are provided to the device. The additional samples may be from the same subject or different subjects. The additional samples may be the same type of sample as the initial sample or different types of samples (e.g., blood, tissue). The additional samples may be provided prior to, concurrently with, and/or subsequent to processing the one or more initial samples on the device. The same and/or different tests or desired criteria may be provided for the additional samples, as opposed to one another and/or the initial samples. The additional samples may be processed in sequence and/or in parallel with the initial samples. The additional samples may use one or more of the same components as the initial samples, or may use different components. The additional samples may or may not be requested in view of one or more detected condition of the initial samples.

In some examples, the system accepts a sample with the aid of a sample collection module, such as a lancet, scalpel, or fluid collection vessel. The system then loads or accesses a protocol for performing one or more processing routines from a plurality of potential processing routines. In an example, the system loads a centrifugation protocol and cytometry protocol. In some examples, the protocol may be loaded from an external device to a sample processing device. Alternatively, the protocol may already be on the sample processing device. The protocol may be generated based on one or more desired criteria and/or processing routines. In one example, generating a protocol may include generating a list of one or more subtasks for each of the input processes. In some examples, each subtask is to be performed by a single component of the one or more devices. Generating a protocol may also include generating the order of the list, the timing and/or allocating one or more resources.

In an example, a protocol provides processing details or specifications that are specific to a sample or a component in the sample. For instance, a centrifugation protocol may include rotational velocity and processing time that is suited to a predetermined sample density, which enables density-dependent separation of a sample from other material that may be present with a desirable component of the sample.

A protocol is included in the system, such as in a protocol repository of the system, or retrieved from another system, such as a database, in communication with the system. In an example, the system is in one-way communication with a database server that provides protocols to the system upon request from the system for one or more processing protocols. In another example, the system is in two-way communication with a database server, which enables the system to upload user-specific processing routines to the database server for future use by the user or other users that may have use for the user-specific processing routines.

In some cases, a processing protocol is adjustable by a user. In an example, a user may generate a processing protocol with the aid of a protocol engine that provides the user one or more options geared toward tailoring the protocol for a particular use. The tailoring may occur prior to use of the protocol. In some examples, the protocol may be modified or updated while the protocol is in use.

With the aid of a protocol, a system processes a sample, which may include preparing the sample, assaying the sample and detecting one or more components of interest in the sample. In some cases, the system performs data analysis with respect to the sample or a plurality of sample after processing. In other cases, the system performs data analysis during processing. In some examples, data analysis is performed on-board--that is, on the system. In other examples, data analysis is performed using a data analysis system that is external to the system. In such a case, data is directed to the analysis system while the sample is being processed or following processing.

A device may be configured to prepare a sample for disposal, or to dispose of a sample, such as a biological sample, following processing or assaying of a sample.

In examples, a sample processing device may be configured to transmit data obtained from a sample. In examples, a sample processing device may be configured to communicate over a network. A sample processing device may include a communication module that may interface with the network. A sample processing device may be connected to the network via a wired connection or wirelessly. The network may be a local area network (LAN) or a wide area network (WAN) such as the Internet. In some examples, the network may be a personal area network. The network may include the cloud. The sample processing device may be connected to the network without requiring an intermediary device, or an intermediary device may be required to connect a sample processing device to a network. A sample processing device may communicate over a network with another device, which may be any type of networked device, including but not limited to a personal computer, server computer, or laptop computer; personal digital assistants (PDAs) such as a Windows CE device; phones such as cellular phones, smartphones (e.g., iPhone, Android, Blackberry, etc.), or location-aware portable phones (such as GPS); a roaming device, such as a network-connected roaming device; a wireless device such as a wireless email device or other device capable of communicating wireless with a computer network; or any other type of network device that may communicate possibly over a network and handle electronic transactions. Such communication may include providing data to a cloud computing infrastructure or any other type of data storage infrastructure which may be accessed by other devices.

A sample processing device may provide data regarding a sample to, e.g., a health care professional, a health care professional location, such as a laboratory, or an affiliate thereof. One or more of a laboratory, health care professional, or subject may have a network device able to receive or access data provided by the sample processing device. A sample processing device may be configured to provide data regarding a sample to a database. A sample processing device may be configured to provide data regarding a sample to an electronic medical records system, to a laboratory information system, to a laboratory automation system, or other system or software. A sample processing device may provide data in the form of a report.

A laboratory, device, or other entity or software may perform analysis on data regarding a sample in real-time. A software system may perform chemical analysis and/or pathological analysis, or these could be distributed amongst combinations of lab, clinical, and specialty or expert personnel. Analysis may include qualitative and/or quantitative evaluation of a sample. Data analysis may include a subsequent qualitative and/or quantitative evaluation of a sample. Optionally, a report may be generated based on raw data, pre-processed data, or analyzed data. Such a report may be prepared so as to maintain confidentiality of the data obtained from the sample, the identity and other information regarding the subject from whom a sample was obtained, analysis of the data, and other confidential information. The report and/or the data may be transmitted to a health care professional. Data obtained by a sample processing device, or analysis of such data, or reports, may be provided to a database, an electronic medical records system, to a laboratory information system, to a laboratory automation system, or other system or software.

### EXAMPLES

### EXAMPLE 1

Examples of individual protein components suitable for use in providing fusion proteins as disclosed herein include (the amino acid sequences are provided using the one-letter code for amino acids):

**TABLE 3**

| Protein Name | Protein Sequence | SEQ ID NO: |
|---|---|---|
| **p50 DNA-binding protein** (fragment from the human NF-kappa-B protein, accession number NP_003989, amino acids 40-366) | | 2 |
| **Tth DNA ligase** (Thermus Thermophilus strain HB8, accession YP_144363.1) | | 3 |
| | | |
| His10-containing leader | mghhhhhhhhhhssghiegras | 4 |
| Flexible glycine-rich sequence | gssgtsgggsggg | 5 |
| **CTF DNA-binding protein** (a hybrid from the murine NFATc1 protein, accession number NP 058071, amino acids 390-506; followed by an alanine spacer; followed by a fragment from the human NF-kappa-B protein, accession number NP 003989, amino acids 249-366) | | 7 |
| **T4 DNA ligase** (accession number NP_049813) | | 8 |
| **Pfu DNA ligase** (Pyrococcus furiosus strain ST04, accession YP_006355162) | | 9 |
| **Pfu C23 DNA ligase** (C-terminal 23-amino acid deletion of Pfu) | | 10 |
| | | |
| HIS-rich leader, p50 DNA-binding protein, glycine-rich linker | | 11 |
| HIS-rich leader, CTF DNA-binding protein, glycine-rich linker | | 12 |

The following provides an example of a fusion protein providing thermostable blunt-end DNA ligase activity:
**p50-Tth ligase fusion,** Theranos construct EE0217, e.g. protein prep RDP0078, consists of a His10-containing leader (mghhhhhhhhhhssghiegras, SEQ ID NO: 4), *p50* (shown below in *italic* text, beginning with *adgpy...* and ending with ... *rkrqk,* SEQ ID NO: 2), a flexible glycine-rich sequence (gssgtsgggsggg, SEQ ID NO: 5), and the Tth DNA ligase (shown below in underlined text, SEQ ID NO: 3). This fusion protein has the following sequence (SEQ ID NO: 1):

The fusion protein sequence SEQ ID NO: 1 presented above is an example of a fusion protein that may be represented as "4-2-5-3" in which "4" represents SEQ ID NO: 4; "2" represents SEQ ID NO: 2; "5" represents SEQ ID NO: 5; and "3" represents SEQ ID NO: 3. Such representations indicate, in a N-terminal to C-terminal orientation, linear fusion proteins comprised of fusions of the amino acid sequence indicated by the numbers (which themselves indicate the corresponding SEQ ID NO:s). Further such representations of fusion proteins are presented below.

An example of a blunt-ended ligation reaction performed at 75°C is provided below. The DNA substrate was a 49-bp duplex DNA made by annealing oligonucleotides EE0139 (SEQ ID NO: 20) and EE0140 (SEQ ID NO: 21). This blunt-ended duplex was capable of making concatamers upon multiple ligation events. The results of the reaction products were separated by size on an agarose gel, as shown in Fig. 1, demonstrate that: (1) Tth ligase alone (lanes 2-3), although it is known to be capable of sealing DNA nicks at 75°C, performed very little or no blunt ligation in these conditions; (2) T4 DNA ligase with an N-terminal p50 fusion (lanes 4-5) also performed very little blunt ligation in these conditions, although there was some evidence of more ligation than for Tth alone. This observation was believed to be surprising given the temperature sensitivity of T4 ligase alone; (3) Tth DNA ligase with an N-terminal p50 fusion (lanes 6-7) demonstrated a much higher level of blunt-end ligation at 75°C. Thus, the results shown in Figure 1 demonstrate that fusion proteins as disclosed herein provide improved blunt-end ligation activity at high temperature. Fusion proteins homologous to SEQ ID NO: 1 are believed to provide improved blunt-end ligation activity at high temperature. Thus, fusion proteins having greater than 90 %, or greater than 95% or greater than 99%, sequence homology to SEQ ID NO: 1 are believed to provide blunt-end DNA ligation activity at high temperature.

### EXAMPLE 2

The fusion protein of SEQ ID NO: 1 (and homologous molecules) is an example of a thermostable blunt-end DNA ligase. Examples of further fusion proteins having similar structural characteristics are listed in the following table (Table 4). It is believed that these further fusion proteins share not only structural characteristics, but also functional characteristics as well; thus, the fusion proteins listed in Table 4 are believed to be thermostable blunt-end ligases. In addition, proteins homologous to the fusion proteins of Table 4 believed to be thermostable blunt-end ligases. The first-listed example, SEQ ID NO: 1, has been discussed above, and results of ligation experiments using SEQ ID NO: 1 are shown in Fig. 1. As discussed above, SEQ ID NO: 1 is a combination of a HIS10-containing leader sequence (SEQ ID NO: 4), the DNA-binding protein p50 (SEQ ID NO: 2), a flexible glycine-rich sequence (SEQ ID NO: 5) and Tth DNA ligase (SEQ ID NO: 3), in which the amino acid sequence SEQ ID NO: 4 is covalently linked to the amino acid sequence SEQ ID NO: 2 which is covalently linked to the amino acid sequence SEQ ID NO: 5 which is covalently linked to the amino acid sequence SEQ ID NO: 3; this combination is termed "4-2-5-3" in the left-most column of Table 4. (The term "4-2-5-3" indicates a linear fusion protein in which sequences SEQ ID NO: 4, SEQ ID NO: 2, SEQ ID NO: 5, and SEQ ID NO: 3 are linked as written, from N-terminal (left) to C-terminal (right).) Further combinations of such components illustrate some of the many possible fusion proteins having features as disclosed herein. The other fusion proteins listed in Table 4 include further combinations of leader sequences (SEQ ID NO: 4) with a DNA-binding protein (e.g., SEQ ID NO: 2 or SEQ ID NO: 7), a flexible glycine-rich sequence (SEQ ID NO: 5), and a DNA ligase (SEQ ID NO: 3, SEQ ID NO: 8, SEQ ID NO: 9, or SEQ ID NO: 10). Thus, exemplary fusion proteins disclosed in Table 4 include an amino acid sequence of a histidine-rich leader sequence, a DNA ligase, and a flexible glycine-rich linker sequence (i.e., SEQ ID NO: 11or SEQ ID NO: 12) covalently linked to a ligase (e.g., SEQ ID NO: 3, SEQ ID NO: 8, SEQ ID NO: 9, or SEQ ID NO: 10). These exemplary fusion proteins are believed to provide yet further thermostable blunt-ended nucleic acid ligases.

**TABLE 4**

| Fusion Protein Formed by SEQ ID NOs: | Protein Sequence | SEQ ID NO: |
|---|---|---|
| 4-2-5-3 | | 1 |
| 4-2-5-8 | | 13 |
| | | |
| 4-2-5-9 | | 14 |
| 4-2-5-10 | | 15 |
| 4-7-5-3 | | 16 |
| | | |
| 4-7-5-8 | | 17 |
| 4-7-5-9 | | 18 |
| 4-7-5-10 | | 19 |

The above-indicated fusion protein sequences provide examples of blunt-end ligases including DNA-ligase portions.

As discussed above, a second amino acid sequence having sequence homology to a first amino acid sequence may differ from the first amino acid by only conservative substitutions; that is, a residue in the second amino acid sequence is either a) identical to the corresponding residue in the first amino acid sequence; b) a member of the same group of amino acids as the corresponding residue in the first amino acid sequence, where the group is based on common side-chain properties as disclosed above: or c) an exemplary or preferred substitution (as identified above in Table 1C above).

For example, suitable fusion proteins include fusion proteins having greater than 90 %, or greater than 95% or greater than 99%, sequence homology or sequence identity to a fusion protein having a composition 4-DNA binding protein-5-DNA ligase (written in the N-terminal to C-terminal orientation), where "DNA binding protein" indicates the amino acid sequence of a DNA binding protein and "DNA ligase" indicates the amino acid sequence of a DNA ligase included in the fusion protein. As discussed above, examples of DNA binding proteins include SEQ ID NO: 2 and SEQ ID NO: 7; examples of DNA ligases include SEQ ID NO: 3, SEQ ID NO: 8, SEQ ID NO: 9, and SEQ ID NO: 10.

### EXAMPLE 3

Further fusion proteins believed to be useful as blunt-end ligases, and believed to be useful as thermostable nucleic acid blunt-end ligases, include fusion proteins as described herein, where the DNA-binding protein is replaced by a RNA-binding protein, and where the ligase comprises an RNA-ligase protein in fusion proteins having a composition 4-RNA binding protein-5-RNA ligase (written in the N-terminal to C-terminal orientation), where "RNA binding protein" indicates the amino acid sequence of a RNA binding protein and "RNA ligase" indicates the amino acid sequence of a RNA ligase included in the fusion protein. In embodiments, the RNA ligase may be a thermostable RNA ligase. Thus, a fusion protein as disclosed herein having RNA ligase activity, and believed to have thermostable RNA ligase activity, comprises a linear polypeptide having a composition including the covalently linked amino acid sequences (in the following N-terminal to C-terminal order) SEQ ID NO: 4, RNA binding protein, SEQ ID NO: 5, RNA ligase.

In addition, suitable RNA ligase fusion proteins include RNA ligase fusion proteins having greater than 90 %, or greater than 95% or greater than 99%, sequence homology to a fusion protein having a composition 4-RNA binding protein-5-RNA ligase. As discussed above, a second amino acid sequence having sequence homology to a first amino acid sequence may differ from the first amino acid by only conservative substitutions; that is, a residue in the second amino acid sequence is either a) identical to the corresponding residue in the first amino acid sequence; b) a member of the same group of amino acids as the corresponding residue in the first amino acid sequence, where the group is based on common side-chain properties as disclosed above: or c) an exemplary or preferred substitution (as identified above in Table 1C above).

### EXAMPLE 4

Proteins having features as disclosed herein include proteins that are homologous to any of the fusion proteins disclosed herein. Such proteins are believed to be suitable for use as nucleic acid ligases, including as thermostable nucleic acid ligases. For example, such proteins are believed to be suitable for use as blunt-end DNA ligases, including as thermostable blunt-end DNA ligases. Such proteins are further believed to be suitable for use as blunt-end RNA ligases, including as thermostable blunt-end RNA ligases.

Proteins that are homologous to any of the fusion proteins disclosed herein may be provided by providing variant proteins having greater than 90 %, or greater than 95% or greater than 99%, sequence homology to a fusion protein disclosed herein. For example, particular proteins that are homologous to any of the fusion proteins disclosed herein may be provided by providing variant proteins having greater than 90 %, or greater than 95% or greater than 99%, sequence homology or sequence identity to SEQ ID NO: 1, SEQ ID NO: 13, SEQ ID NO: 14, SEQ ID NO: 15, SEQ ID NO: 16, SEQ ID NO: 17, SEQ ID NO: 18, or SEQ ID NO: 19 (i.e., having greater than 90 %, or greater than 95% or greater than 99%, sequence homology or sequence identity to a fusion protein of Table 4).

Fusion proteins as disclosed herein include a composition having the general form of a linear molecule having a histidine-rich leader sequence covalently linked to a nucleic acid binding protein sequence which is covalently linked to a flexible glycine-rich sequence which is covalently linked to a nucleic acid ligase sequence. In embodiments, such fusion proteins may be proteins including one or more amino acid substitutions, insertions, deletions, or additions as compared to a fusion protein sequence provided as a sequence listing herein. In embodiments, such fusion proteins may be proteins including one or more amino acid substitutions, insertions, deletions, or additions as compared to a histidine-rich leader sequence, a nucleic acid binding protein sequence, a flexible glycine-rich sequence, or a nucleic acid ligase sequence provided as a sequence listing herein.

For example, homologous fusion proteins having features disclosed herein may include histidine-rich leader sequences different than, while homologous to, SEQ ID NO: 4; e.g., having histidine-rich leader sequences having greater than 90 %, or greater than 95% or greater than 99%, sequence homology or sequence identity to SEQ ID NO: 4. It will be understood that such homologous histidine-rich leader sequences may include the same number, or may include a greater number, or may include a smaller number, of amino acid residues than SEQ ID NO: 4.

For example, homologous fusion proteins having features disclosed herein may include flexible glycine-rich sequences different than, while homologous to, SEQ ID NO: 5; e.g., having flexible glycine-rich sequences having greater than 90 %, or greater than 95% or greater than 99%, sequence homology or sequence identity to SEQ ID NO: 5. It will be understood that such homologous flexible glycine-rich sequences may include the same number, or may include a greater number, or may include a smaller number, of amino acid residues than SEQ ID NO: 5.

For example, homologous fusion proteins include fusion proteins having compositions homologous to any of the proteins described herein as 4-2-5-3, 4-2-5-8, 4-2-5-9, and 4-2-5-10, where the sequence "2" is replaced by a sequence having greater than 90 %, or greater than 95% or greater than 99%, sequence homology or sequence identity to SEQ ID NO: 2; or where the sequence "3", or "8", or "9", or "10" is replaced by a sequence having greater than 90 %, or greater than 95% or greater than 99%, sequence homology or sequence identity to SEQ ID NO: 3, SEQ ID NO: 8, SEQ ID NO: 9, or SEQ ID NO: 10. In embodiments, both the sequence "2" is replaced by a homologous sequence and the sequence "3", or "8", or "9", or "10" is replaced by a homologous sequence.

In addition, homologous fusion proteins include fusion proteins having compositions homologous to any of the proteins described herein as 4-7-5-3, 4-7-5-8, 4-7-5-9, and 4-7-5-10, where the sequence "7" is replaced by a sequence having greater than 90 %, or greater than 95% or greater than 99%, sequence homology or sequence identity to SEQ ID NO: 7; or where the sequence "3", or "8", or "9", or "10" is replaced by a sequence having greater than 90 %, or greater than 95% or greater than 99%, sequence homology or sequence identity to SEQ ID NO: 3, SEQ ID NO: 8, SEQ ID NO: 9, or SEQ ID NO: 10. In embodiments, both the sequence "7" is replaced by a homologous sequence and the sequence "3", or "8", or "9", or "10" is replaced by a homologous sequence.

While the above is a complete description of the preferred embodiment as described herein, it is possible to use various alternatives, modifications and equivalents. Therefore, the scope of the present invention should be determined not with reference to the above description but should, instead, be determined with reference to the appended claims.

Any feature, whether preferred or not, may be combined with any other feature, whether preferred or not. The appended claims are not to be interpreted as including means-plus-function limitations, unless such a limitation is explicitly recited in a given claim using the phrase "means for." It should be understood that as used in the description herein and throughout the claims that follow, the meaning of "a," "an," and "the" includes plural reference unless the context clearly dictates otherwise. Also, as used in the description herein and throughout the claims that follow, the meaning of "in" includes "in" and "on" unless the context clearly dictates otherwise. Finally, as used in the description herein and throughout the claims that follow, the meanings of "and" and "or" include both the conjunctive and disjunctive and may be used interchangeably unless the context expressly dictates otherwise. Thus, in contexts where the terms "and" or "or" are used, usage of such conjunctions do not exclude an "and/or" meaning unless the context expressly dictates otherwise.

## Claims

1. A method for performing a thermostable blunt-end DNA ligase reaction, comprising: combining DNA molecules to be ligated and a thermostable blunt-end DNA ligase in a solution, wherein said thermostable blunt-end DNA ligase comprises an amino acid sequence selected from the group of amino acid sequences consisting of SEQ lD NO: 1, SEQ lD NO: 13, SEQ lD NO: 14, SEQ lD NO: 15, SEQ lD NO: 16, SEQ lD NO: 17, SEQ lD NO: 18, and SEQ lD NO: 19, and wherein the thermostable blunt-end DNA ligase reaction is performed at a temperature of 60 °C or higher.

2. The method of claim 1, wherein the thermostable blunt-end DNA ligase reaction is performed at a uniform temperature.

3. The method of claim 1, wherein the thermostable blunt-end DNA ligase reaction is performed at a temperature of 60 °C to 65 °C.

4. The method of claim 1, wherein the thermostable blunt-end DNA ligase reaction is performed at a temperature of 75 °C.

5. The method of claim 1, wherein the thermostable blunt-end DNA ligase reaction comprises temperature cycling.

6. The method of claim 1, wherein the thermostable blunt-end DNA ligase reaction comprises temperature cycling from 94 °C to 60 °C.

7. The method of claim 1, wherein the thermostable blunt-end DNA ligase reaction comprises two or more cycles of temperature cycling.

8. The method of claim 1, further comprising multiple ligation events effective to provide concatamers.

## Patentansprüche

1. Verfahren zum Durchführen einer thermostabilen DNA-Ligasereaktion mit stumpfen Enden, das Folgendes umfasst: Kombinieren von zu ligierenden DNA-Molekülen und einer thermostabilen DNA-Ligase mit stumpfen Enden in einer Lösung, wobei die thermostabile DNA-Ligase mit stumpfen Enden eine Aminosäuresequenz umfasst, die aus der Gruppe von Aminosäuresequenzen ausgewählt ist, die aus SEQ ID NO: 1, SEQ ID NO: 13, SEQ ID NO: 14, SEQ ID NO: 15, SEQ ID NO: 16, SEQ ID NO: 17, SEQ ID NO: 18 und SEQ ID NO: 19 besteht, und wobei die thermostabile DNA-Ligasereaktion mit stumpfen Enden bei einer Temperatur von 60 °C oder höher durchgeführt wird.

2. Verfahren nach Anspruch 1, wobei die thermostabile DNA-Ligasereaktion mit stumpfen Enden bei einer einheitlichen Temperatur durchgeführt wird.

3. Verfahren nach Anspruch 1, wobei die thermostabile DNA-Ligasereaktion mit stumpfen Enden bei einer Temperatur von 60 °C bis 65 °C durchgeführt wird.

4. Verfahren nach Anspruch 1, wobei die thermostabile DNA-Ligasereaktion mit stumpfen Enden bei einer Temperatur von 75 °C durchgeführt wird.

5. Verfahren nach Anspruch 1, wobei die thermostabile DNA-Ligasereaktion mit stumpfen Enden einen Temperaturzyklus umfasst.

6. Verfahren nach Anspruch 1, wobei die thermostabile DNA-Ligasereaktion mit stumpfen Enden einen Temperaturzyklus von 94 °C bis 60 °C umfasst.

7. Verfahren nach Anspruch 1, wobei die thermostabile DNA-Ligasereaktion mit stumpfen Enden zwei oder mehr Zyklen von Temperaturzyklen umfasst.

8. Verfahren nach Anspruch 1, das ferner mehrere Ligationsereignisse, die wirksam sind, um Konkatamere bereitzustellen, umfasst.

## Revendications

1. Procédé destiné à effectuer une réaction d'ADN ligase à extrémités franches thermostable, comprenant : la combinaison de molécules d'ADN à ligaturer et d'un ADN ligase à extrémités franches thermostable dans une solution, ledit ADN ligase à extrémités franches thermostable comprenant une séquence d'acides aminés choisie dans le groupe de séquences d'acides aminés constitué de SEQ ID N° : 1, SEQ ID N° : 13, SEQ ID N° : 14, SEQ ID N° : 15, SEQ ID N° : 16, SEQ ID N° : 17, SEQ ID N° : 18 et SEQ ID N° : 19, et la réaction d'ADN ligase à extrémités franches thermostable étant effectuée à une température de 60 °C ou plus.

2. Procédé selon la revendication 1, dans lequel la réaction d'ADN ligase à extrémités franches thermostable est effectuée à une température uniforme.

3. Procédé selon la revendication 1, dans lequel la réaction d'ADN ligase à extrémités franches thermostable est effectuée à une température de 60 °C à 65 °C.

4. Procédé selon la revendication 1, dans lequel la réaction d'ADN ligase à extrémités franches thermostable est effectuée à une température de 75 °C.

5. Procédé selon la revendication 1, dans lequel la réaction d'ADN ligase à extrémités franches thermostable comprend un cyclage en température.

6. Procédé selon la revendication 1, dans lequel la réaction d'ADN ligase à extrémités franches thermostable comprend un cyclage en température de 94 °C à 60 °C.

7. Procédé selon la revendication 1, dans lequel la réaction d'ADN ligase à extrémités franches thermostable comprend deux cycles ou plus de cyclage en température.

8. Procédé selon la revendication 1, comprenant en outre de multiples événements de ligature efficaces pour fournir des concatémères.
